(19) Europäisches Patentamt

European Patent Office

Office européen des brevets

(11) Publication number: 0 308 716

A2

(12) **EUROPEAN PATENT APPLICATION**

(21) Application number: 88114516.3

(22) Date of filing: 06.09.88

(51) Int. Cl.⁴: **C12N 9/72 , C12N 15/00 , A61K 37/54**

Claims for the following Contracting States: ES + GR.

(30) Priority: 07.09.87 GB 8721023

(43) Date of publication of application:
**29.03.89 Bulletin 89/13**

(84) Designated Contracting States:
**AT BE CH DE ES FR GB GR IT LI LU NL SE**

(71) Applicant: **GRUPPO LEPETIT S.P.A.**
**23, Via G. Murat**
**I-20159 Milano(IT)**

(72) Inventor: **Cassani, Giovanni**
**3, Via Vittadini**
**I-27100 Pavia(IT)**
Inventor: **Blasi, Francesco**
**298, Via Posillipo**
**I-80123 Napoli(IT)**
Inventor: **Robbiati, Federico Maria**
**141, Via Porpora**
**I-20131 Milano(IT)**
Inventor: **Nolli, Marialuisa**
**34, Via C. Riboldi**
**I-27100 Pavia(IT)**

(54) **Modified plasminogen activators.**

(57) This invention relates to a method for producing Growth Factor-like domain (GFD) altered, single chain urokinase (scuPA)-derived, glycosylated plasminogen activators (hereinbelow referred to also as GASGAs). The compounds of the invention are preferably prepared by genetic engineering of the uPA coding DNA and possess improved pharmacological properties.

EP 0 308 716 A2

## MODIFIED PLASMINOGEN ACTIVATORS

This invention relates to a method for producing Growth Factor-like domain (GFD) altered, single chain urokinase (scuPA)-derived, glycosylated plasminogen activators (hereinbelow referred to also as GASGAs). More particularly, it relates to a method for producing GASGAs which comprises recovering the uPA gene from a human genomic library, inserting it into an expression vector, modifying the plasmid and altering the GFD of the uPA gene, introducing the plasmid vectors so obtained into an animal cell to produce therewith transformants and recovering GFD altered, scuPA-derivated, glycosylated plasminogen activators from the transformed animal cells.

A further object of the invention is represented by human urinary plasminogen activator derivatives having the Growth factor-like domain (GFD) altered. More particularly, the present invention encompasses single or double chain human urinary plasminogen activator derivatives having the region between aminoacid 9 and aminoacid 50 of the sequence of human scu-PA (c.f. for instance the sequence of uPA published in EP-A-92182), preferably, between aminoacid 9 and aminoacid 45, and most preferably between aminoacid 19 and aminoacid 32, altered.

Urokinase (uPA) is a plasminogen activator, obtainable from human urines, which is used in the treatment of various forms of thrombosis or embolic diseases. Urokinase production has been based on the purification of the enzyme essentially from human urine. With the development of cell culture technology and the availability of specific anti-urokinase antibodies (monoclonal as well as polyclonal antibodies), it has been shown that uPA is an activation product of cell-produced pro-uPA (Wun et al., 1982, J. Biol. Chem. 257,7262-7268; Nielsen et al., 1982, Biochemistry 21, 6410-6415). Pro-uPA in a single-chain protein, while uPA is a two-chain protein. Conversion of pro-uPA into uPA depends on the cleavage of a single peptide bond of pro-uPA (Verde et al., 1984; Proc. Natl. Acad. Sci. USA 81; 4727-4731; (1984); Günzler et al., Hoppe S. Zschrp Physiol. Chem. 363, 1155-1165, 1982). This specific cleavage of single chain pro-uPA results in over 50 fold increase in specific activity and is obtained with proteolytic enzymes such as trypsin or plasmin. In particular, plasmin appears to be responsible of this conversion also in vivo.

An inactive form of plasminogen activator was identified in human embryonic kidney cell cultures by C. Nolan already in 1977 et al., (Biochim Biophys Acta 1977; 496:384-400). This substance, that the authors considered to be a pro-activator form of urokinase, was activated by trypsin without change in molecular weight and, once activated, was inhibited by rabbit antibody to human urokinase. The inactive "pro-activator" was separated from the active form by means of an affinity chromatography on benzamidine-Sepharose.

Stoppelli M.P. et al. described the isolation and characterization of a uPA amino terminal fragment (ATF) spanning from the aminoacid residue in position 1 to that in position 135 in Proc. Natl. Acad. Sci. USA, 1985, 82, 4939-4943.

A single-chain plasminogen activator having molecular weight of about 56,000, a specific activity of 40,000-50,000 CTA units/mg on a fibrin plate and with a high affinity for fibrin is described in European patent 40238.

European Patent Application Publ. No. 92182 deals with the preparation of urokinase derivatives via recombinant DNA technology. In particular it discloses the purification and characterization of low molecular weight urokinase (about 30,000 daltons) obtained by recombinant DNA techniques in a procariotic host (E.coli).

A method for producing glycosylated single-chain urokinase in animal cells by recombinant cDNA technology using a cDNA obtained from the mRNA of an established human kidney-derived cell line is disclosed in European Patent Application publication No. 154272.

The cloning of human uPA gene has been described by Riccio et al. (Nucl. Acid Res., 1985, 13, 2759-2771). The coding region is subdivided in eleven exons separated by ten introns. They report that functional domains in the protein can be reconducted to different exons or groups of exons. In particular, a certain level of homology between the aminoacid sequence from 10 to 50, which is mostly synthetized by exon IV, and epidermal growth factor (EGF) and the alpha-type transforming factor is found.

This growth factor-like domain appears to be involved in the protein (uPA/scu-PA) recognition and binding by cell-membrane specific receptors (Stoppelli et al., Proc. Natl. Acad. Sci, USA, 1985, 82, 4939-4943 and Appella et al., J. Biol.. Chem., 1987, 262, 4437-4440).

The GFD-altered urokinase like derivatives of the invention (GASGAs) are scu-PA/uPA derivatives wherein the protein GFD has been altered by one of the known means to obtain new derivatives with a prolonged duration of action when compared to uPA. This prolonged action may be shown both in in vitro

2

tests and in in vivo experiments (in particular, on conventional animal models which are known to be predictive of activity in man). Considering the short half-life of uPA in vivo the need for compounds having the properties of the compounds of the invention is evident to the man skilled in the art.

Another group of compounds of the invention is represented by those compounds wherein one or more copies of the GFD sequence are inserted, preferably immediately downstream or upstream from the "natural" GFD.

Methods so far known to modify a protein in a certain specific sequence includes "direct" protein modification, e.g. by chemical means, and "indirect" protein modification, e.g. by genetic engineering manipulations of the coding region of the protein. Chemical modifications includes selectively reacting "native" scu-PA or u-PA with derivatizing reagents for one or more of the aminoacid functions in the region between aminoacid 9 and aminoacid 50, preferably between aminoacid 9 and aminoacid 45 and most preferably between aminoacid 19 and aminoacid 32. The derivating agents are known per se. For instance, the basic aminoacids, like lysine or arginine, a reagent capable of forming amide bonds or other organic bonds with an amino function such as acetic anhydride, 1,2-cyclohexandione and phenylglioxal can be used, while for acid aminoacids, such as aspartic acid, esterification may be the most convenient derivatization. If necessary, the selective reaction conditions can be coupled to the protection of other labile function on the protein by means of known per se protecting agents.

A greatly preferred modification method is however represented by the genetic engineering of the protein coding region and in particular of the DNA and/or mRNA regions coding for the protein GF domain, i.e. the regions coding for the aminoacids between 9 and 50, preferably between 9 and 45 and most preferably between 19 and 32, in order to introduce modifications in the final protein so that it does not bind to its specific receptor, such as the cell receptor on U 937 monocytes (c.f. Stoppelli M.p. et al., Proc. Natl. Acad. Sci, USA, 82, 4939-4943, 1985).

A preferred modification is a genetic engineering modification of the uPA coding genomic DNA between 1027 bp and 1784 bp (assuming the mRNA transcription initiation site as bp 1).

Genetic modifications of the genetic region coding for the binding region of "native" protein can be carried out on genomic DNA or cDNA. They include causing site-directed deletions, mutations and insertions on the nucleic acid sequence. The main result of these modifications is the impairment of the receptor-binding capacity of scu-PA or uPA, while maintaining its enzymatic capability unaltered. Occasionally, and indeed frequently, a new aminoacid can occur in the sequence of the modified protein as a result of said modifications. For instance, when a deletion between the PstI site in intron C (i.e. the intron between exons III and IV of uPA gene) and the Bgl II site in intron D (i.e. the intron between exons IV and V) is carried out, a GASGA is obtained wherein the deletion of the aminoacid sequence between $Ser_9$ and $Asp_{45}$ is accompanied by the introduction in its place of a new aminoacid, namely tyrosine, coded by the triplet which originates by the ligation process following the deletion.

A preferred embodiment of the invention is represented by a modification process which includes deleting the region corresponding to exon IV in a uPA genomic or cDNA or in a hybrid uPA genomic DNA-cDNA. When this is done on a genomic sequence, it is also possible and sometimes even convenient to cut the DNA sequence in the intron regions relatively close to exon IV. This can be done by several known per se procedures. A preferred procedure is represented by the introduction of unique restriction sites in the introns surrounding exon IV in a vector containing the whole uPA ORF downstream from a promoter, thus obtaining two vector derivatives differing only for the presence of a new site in intron C or in intron D. Restriction digestion at these new sites as well as at the third one in this region which is common to the two plasmids and religation of the resulting plasmid fragments produces, among the others, a new plasmid wherein the region upstream from the new site in intron C has been fused to the region downstream from the new site in intron D, i.e. a sequence missing virtually all the region corresponding to the uPA GFD. A further preferred modification process comprises transforming by known per se techniques the Pst I site in intron C of the uPA coding region into a SalI site and transforming the BglII site in intron D into a XhoI, deleting the region between these two sites with the appropriate restriction enzymes and linking the two fragments together to obtain a new uPA coding sequence which codes for a GASGA derivative wherein the aminoacid sequence between $Ser_9$ and $Asp_{45}$ is replaced by a Tyr unit. More particularly, the resulting plasmid is missing the aminoacid sequence from $Ser_9$ to $Lys_{23}$ and from $Phe_{25}$ to $Asp_{45}$ while $Tyr_{24}$ which is originally codified by a TAC codon has been substituted by a Tyr residue which is codified by the TAT codon originated by the fusion of the ORF of exon III to that of exon V.

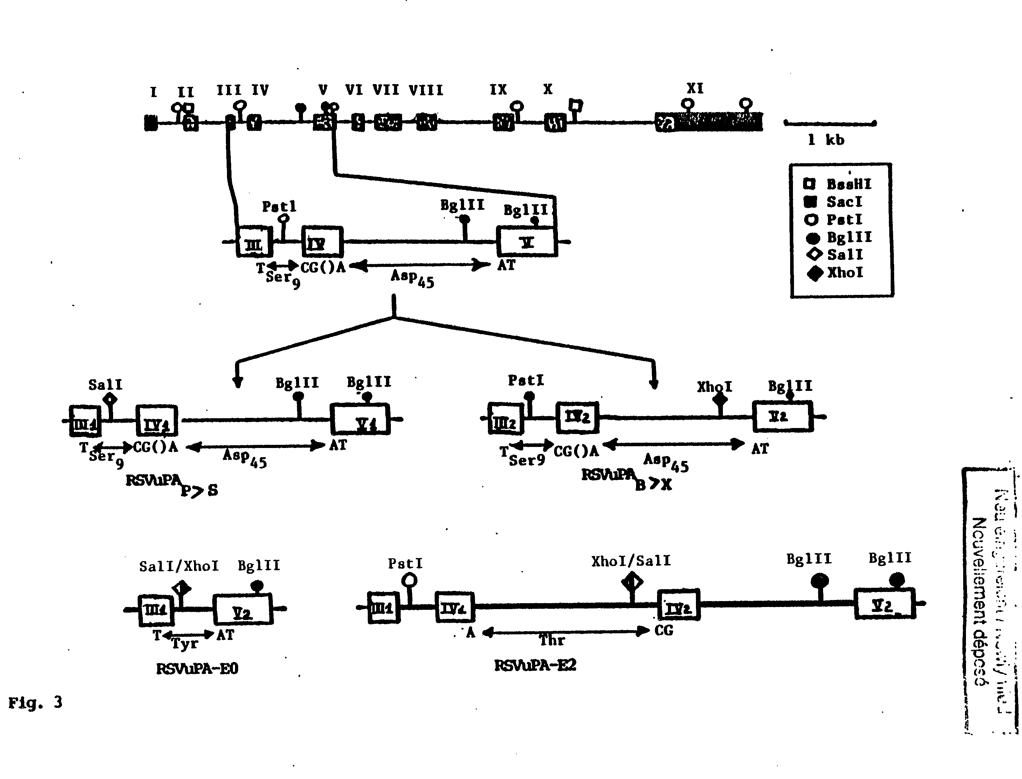

Preferably, the above process is carried out on a vector which contains the uPA ORF (open reading frame) so that a GASGA expressing vector is obtained directly. Alternatively, a deletion mutant can also be obtained from a uPA coding plasmid having unique site introduced downstream from exon IV as described above, (e.g. a XhoI site) by deleting larger DNA portions upstream and downstream from exon IV and replacing these sequences with a fragment which reintroduces a part of the deleted sequence which does not include exon IV. More particularly, a vector containing the uPA ORF and unique restriction sites as described above is treated with the enzyme which is specific for the unique site introduced downstream from exon IV (e.g. XhoI) and with another enzyme which is specific for another unique site upstream from exon IV (e.g. HindIII which is already present in intron B, i.e. the intron preceeding exon IV). The resulting plasmid fragment which misses exon III and IV is linked by appropriate cohesive or blunt ends to a fragment containing only exon III (but not exon IV) in the proper orientation in order to maintain the correct reading frame. The obtained vector is a GASGA expression vector coding for a protein which is a GASGA derivative of the invention. In a preferred embodiment of this method, a uPA expressing vector containing the ORF derived from the uPA gene is modified by inserting a XhoI site in place of the BglII site present in intron D (Fig. 3) followed by digestion with XhoI and HindIII. A DNA fragment containing most of the original plasmid but lacking essentially the sequence corresponding to exons III and IV is obtained and isolated. This fragment is linked to a HindIII-SalI fragment spanning from intron B to intron C, and thus containing exon III, to obtain a GASGA expression vector of the invention wherein there is a uPA coding sequence which lacks essentially the DNA sequence corresponding to exon IV, in the proper orientation frame and thus capable of codifying a GASGA protein of the invention. The HindIII-SalI fragment mentioned above, is obtained by HindIII digestion following a SalI linkering to the ends of a PstI fragment essentially containing exon III which is obtained by PstI treatment of a vector containing the uPA coding sequence of genomic origin. The obtained expression vector codes for a GASGA that misses the amino acids from $Ser_9$ to $Lys_{23}$ and from $Phe_{25}$ to $Asp_{45}$ and in which $Tyr_{24}$ codes by a TAC codon, has been replaced by a Tyr coded by the TAT codon originating from the fusion of the ORF of exon III with that of exon V.

As mentioned above, a uPA derivative of the invention (GASGA) can be obtained also with insertions in the GFD of "natural" uPA so that its uPA receptor binding capacity is substantially reduced or completely impaired. A preferred method includes modifying a uPA coding gene or cDNA or a hybrid uPA gene/cDNA by genetic engineering. It is known in fact that gene mutagenesis can be obtained with a number of physical and chemical agents such as ionizing radiations, U.V. or DNA precursor analogues. Anyway, the preferred way is to modify directly the coding sequence by genetic engeneering e.g. by using site directed mutagenesis in M13-derived vectors (P. Carter, Improved oligonucleotide site-directed mutagenesis using M13 vectors, Nucl. Acid Research 1985, 13, 4431) or Polymerase Chain Reaction (PCR). The coding sequence can be modified either on the same vector to be transfected or on a different vector and subsequently a suitable fragment transferred to the transfection vector. The order of these operations depends on the specific substrates involved and is determined by the man skilled in the art on the basis of the usual practice.

A restriction site (ScaI) is present in the DNA sequence coding for the portion of uPA implied in the binding to the uPA receptor. It is possible to open it and to insert a DNA stretch that neither stops the protein translation nor changes the frame of reading. Preferentially said DNA stretch will contain a new restriction site to facilitate the identification of the obtained mutants. The number of base-pairs added can be three or a multiple thereof. This can be obtained either directly with a DNA stretch of the desired length or by multiple steps such as by inserting a larger stretch which is then reduced to the desired length by appropriate restrictions.

The obtained coding sequence can codify for a protein with an altered uPA receptor binding region e.g. because of an alteration in the beta-sheets, alpha-helicals of the "original" uPA secondary structure, such as introduction of turns in it, spacing of the aminoacid residues implicated in the binding, modification of the hydropaticity profile or a combination of these effects.

Briefly, the following operations are carried out:
- opening an expression vector containing the human uPA gene either by partial or total digestion with ScaI;
- inserting a new restriction site by a DNA linker of known sequence;
- modifying said restriction site to obtain an insertion of three base pairs or a multiple thereof;
- transfecting mammalian cells with this vector in the presence of a plasmid carrying the resistance to a selection agent;
- selecting the resistant clones and, among them, those producing GASGAs in the medium;
- collecting the medium and purifying the protein from it.

According to the process of the invention, it is also possible to insert a multiple of the GF domain in a "native" scu-PA or uPA by inserting one, two or more replicas of the uPA DNA region between 1027 bp and 1784 bp in the uPA coding sequence, preferably sequentially with the original 1027 to 1784 bp GFD coding region (i.e., essentially exon IV).

These GASGAs derivatives with poly-GF domain are expected to have improved and a more selective binding ability to the uPA receptor when compared with the native molecule.

A vector coding for GASGAs having the GF-domain altered (and thus no longer capable of binding the uPA-receptor) will belong to the series identified below as "E0" while the multiple GFD-containing GASGAs expressing vectors will be identified as belonging to the "E2", "E3", "E4", etc. series depending on the number of the contained GFD units. GASGAs derivatives will be identified accordingly.

The present invention encompasses the GASGAs derivatives as well as the allelic forms thereof having the characteristic impairment or substantial reduction of the uPA receptor binding activity. Allelic forms can be obtained by modifying "native" scu-PA and/or PA, preferably by modifying the uPA coding region and expressing this modified sequence in mammalian cells. Moreover, the present invention encompasses Gf-domain altered plasminogen activators having other regions modified, preferably the region which is exposed to plasmin cleavage. Examples of scu-PA derived plasminogen activators having a region modified in order to make it more resistant to its conversion to uPA are the $Lys_{135}$ $Lys_{136}$ and $Arg_{156}$ $Lys_{158}$ modified derivatives described in EP-A-200451 or the $Lys_{137}$, $Phe_{157}$ modified derivatives described in EP-A-210279.

These compounds are modified in the region between aminoacid 9 and aminoacid 50, preferably between aminoacid 9 and 45, and most preferably between aminoacid 19 and aminoacid 32 by means of site-directed mutations on the cDNA sequence or the corresponding DNA sequence. The resulting coding sequence inserted in an expression vector is used to transform suitable animal cells to produce the desired GfD-altered plasminogen derivatives.

These GFD-altered, scu-PA derived plasminogen activators, having also the region usually exposed to plasmin-type cleavage modified to confer a certain resistance to proteolytic cleavage, may possess an increased duration of action when compared with the GFD unaltered starting compounds, and/or can be administered in lower dosages to obtain the desired effect. The order and methodology of these alterations may not be critical, provided that the feature of non-binding to the uPA receptor is maintained. In particular, the GFD-mutation can be introduced after the mutation in the plasmin-sensible region or viceversa, the GFD-mutation can be introduced before the mutation in the plasmin-sensible region.

The DNA fragment of human genomic origin containing the uPA coding region which is used in an embodiment of the present invention is obtained by a human genomic library in a suitable vector, such as a library prepared in bacteriophage lambda or in cosmids according to known per se techniques, after screening the library with the proper DNA probe represented by a portion of the gene itself or a synthetic DNA fragment of a suitable lenght prepared on the basis of the known coding sequence or a CDNA or mRNA derived probe.

Conveniently, this uPA coding fragment is the 7.3 kb fragment obtained by partial SmaI digestion of the cloned DNA.

A DNA segment like this will be referred to in the description and claims as "open reading frame" (ORF), to mean a genomic DNA sequence including the transcription-translation unit of uPA mRNA, possibly interrupted by introns and preferably including 5′ and 3′ untranslated regions or a part thereof which confers translation efficiency or mRNA stability. A preferred example of ORF is a sequence defined as above which is interrupted by 10 introns.

Expression vectors that can be used in the method of the invention are any of the known vectors used to transfect eukaryotic cells and in particular mammalian cells. Essentially they include a sequence which regulates the mRNA translation, such as a promoter, and a polyadenylation site. Sometimes, they include a eukaryotic origin of replication.

In particular, the promoter region may be any of the known ones which may be used in animal cells genetic engineering. Heterologous promoters may be obtained from mammalian virus such as retroviruses (e.g. RSV), SV 40 (early or late) and adenovirus (early or late).

Alternatively, regulable promoters of animal or human origin may be used that may be induced to transcribe over base levels by physical, chemical, biological inducers. Examples of these regulable promoters are these from mouse or human metallothioneins I and II, heat shock proteins, etc. which are found in reference publications such as Hamer et al., Eukaryotic Viral Vectors, Y. Gluzman ed., 1983, 7-12, Mayo et al, Cell, 29, 99-108 (1982) and Korin et al., Nature, 308, 513-519 (1984).

In addition to those regulable promoters it has been also found that the human uPA promoter region or a derivative thereof may be suitably used as an efficient promoter for expressing a gene put under its control in mammalian cells. The human uPA promoter region is obtained from a human genomic library (see above) by SmaI digestion and contains about 2.38 kb, from about -2353 bp to about + 29 bp on the human uPA gene. Not only this "native" human uPA promoter can be used to express a gene in a suitable mammalian cell system but any of its derivatives obtained by deletion, insertion, substitution or shuffling which maintain or even possess an improved regulatory funtion can suitably be used in the method of the invention. In particular, deletion derivatives of the uPA promoter region may be, at least in some instances, preferred. Representative examples of deletion derivatives are those obtained by treating the uPA promoter with OxaNI or EcoRV and subsequent ligation of the obtained fragments. In particular by digesting with OxaNI a deletion derivative is obtained which misses a 0.6 kb fragment between about -1827 bp and -1202 bp, where +1 is the first base transcribed in the uPA mRNA, while by digesting with OxaNI and EcoRV a deletion derivative is obtained which misses a 1.29 kb fragment between about -1827 bp and -537 bp. It has also been found that these uPA promoter deletion derivatives are capable of driving the expression of the regulated gene with comparable and even higher yields than usual viral promoters, such as RSV, which are commonly considered very efficient promoter systems. As for the polyadenylation site, it may be any of the known ones, such as that associated with the promoter region native to the uPA gene, or from a eukaryotic or viral gene. Once an expression cassette has been prepared containing a promoter region, a trascription initiator and, conveniently, a polylinker, the chosen gene sequence can be inserted in it for transformation into an animal cell.

In general, the specific order in which the parts are brought together is not critical, so that the flanking regions might be first bound to a replication system including a selectable marker or other regions, such as enhancers, transcriptionl regulatory regions, or the like, prior to insertion of the gene. The manner in which the various fragments are brought together will depend on a number of factors related to ease of construction, choice of restriction sites, availability of particular fragments, and the like, which ultimately are within the choice capability of the man skilled in the art.

The choice of the replication system depends, to a certain extent, on whether a transient or stable expression is desired. For transient expression, episomal elements based on viral sequence, such as SV40, containing an origin of replication are used. Stable expression can be achieved using other episomal elements (e.g. bovine papilloma virus based vectors) or sequences integrated into the genome. A number of these viral sequences have been joined to selectable markers, such as the genes gpt, neo and dhfr. These markers allow for selection of cells containing the vector and some of them for amplification of the integrated foreign sequences.

To ultimately enhance the production of the desired substance, it may be convenient to co-transform the cells with a first construct containing the GASGA expression cassette and a second independent construct containing the selectable marker. Alternatively, the gene may be amplified by preparing a tandem construct where the GASGA cassette is in tandem with an expression cassette of a gene such as dhfr or metallothionein. The host cells may be any animal cell, preferably a mammalian or avian cell, which can produce a GASGA in a recoverable amount upon transformation with a GASGA expression vector. Examples of these cells, which expressely include cells of human origin, encompass the known and established cell lines which may or may not produce pro-upA in recoverable amounts before transformation, such as LB6, a mouse fibroblastoid cell line derived from parental L cells (Corsaro C.M. and Pearson L.M. Enhancing the efficiency of DNA-mediated gene transfer in mammalian cells, Somatic cell genetics 7, 603-616, 1981); CHO, chineese hamster ovary (Kao F.T., Puck F., Proc. Natl. Acad. Sci., 60, 1275-1281, 1968) and A 431 human epidermoid carcinoma cell line (Fabricant R.N., De Larco J. E., and Todaro G.F., Nerve growth factor receptors on human melanoma cells in culture, Proc. Natl. Acad. Sci. USA 74, 565-569, 1977). The first two cell lines mentioned above (LB6 and CHO) do not produces pro-upA while the last mentioned one (A 431) produces it. In general, however, established cells lines such as CHO, COS, LTK, Hela and CN-1 may be suitably used in the process of the invention.

Upon repeated recloning of a GASGA producing mono-layer CHO cell line, derived cell lines can be isolated from the medium which retain the uPA production capability and which are also capable of self-replication in suspension. The advantage of such cell lines in terms of easiness of cultivation and higher production yields per fermentation mass unit are evident to the man skilled in the art. These GASGA producing CHO cell lines capable of growth in suspension in a cultivation medium represent a preferred embodiment of the present invention.

The expression vector may be introduced into the host by any convenient means such as microinjection, DEAE-dextran mediated transfection, calcium phosphate precipitated DNA transfection and electroporation (see Bonerji J. et al., Cell 33, 729-740 (1983); Graham F.L. and Van der Eb A.J., Virology, 52, 456-467 (1983) and Potter H. et al., Proc. Natl. Acad. Sci. USA, 81, 7161-7165 (1984)).

After transfection, the cells are grown in an appropriate medium, such as Dulbecco's modified Eagle medium (DMEM) containing 10% to 20% inactivated fetal calf serum, in which these cells are stably maintained and then the appropriate selection agent e.g. an antibiotic, is introduced.

The selected cells are repeatedly cloned and their production of GASGAs is evaluated by the usual assays such as the fibrinolytic assay or the immunoamidolytic assay (IAA).

This assay must be conducted using a monoclonal antibody specific for scu-PA and or uPA which does not recognize or bind specifically to the protein GFD. Suitable monoclonal antibodies are those which recognize the uPA B-chain without recognizing the A-chain, such as those described by Herion P. et al. in Bioscience Reports 1, 885-892 (1981), Salerno M.G. et al, Proc. Natl. Acad. Sci, USA, 1984, 81, 110-114, or Kaltoft et al., Proc. Natl. Acad. Sci. USA, 1982, 79, 3720-3723 or MAB 5B4 which was described by Nolli et al. in Thromb. Haemostas. 1986, 56, 214-218 and which binds the uPA "Kringle" region, without binding to the GFD region.

The isolated GASGA producing clones are then mass cultivated to produce substantial amounts of GASGAs. In mass cultivation, a preferred embodiment is represented by the addition of aprotinin. The result of the addition of this substance in the original medium or during cultivation is that pro-uPA is recovered in higher amounts. Typically aprotinin is added at a concentration of 10-50 IU/ml and most preferably at 20-25 IU/ml.

Depending upon whether the leader sequence has been retained, and the product is capable of secretion, the medium may be continuously or repetitively exchanged and the GASGAs isolated from the medium. If the leader has not been retained and the product is maintained intracellularly, the cells may be harvested, killed and lysed, and the product isolated. For isolation and purification, the usual techniques may be applied such as ion exchange or affinity chromatography, HPLC and reverse-phase HPLC. A convenient affinity chromatography purification involves the use of a anti-scu-PA or uPA monoclonal antibody which does not recognize or bind the protein GFD such as those which bind the uPA B-chain without binding the A-chain or binding to the A-chain only in the "Kringle" region (see above). Conveniently, the immunoadsorbent described by Herion P. and Bollen A. in Bioscience 1983, 3, 373-379 can be used as well as MAB 5B4 (Nolli et al., see above).

A convenient procedure to ascertain that the obtained compounds do not in fact bind to the uPA GFD is represented by an immunoamidolytic assay (c.f. Corti et al, Thromb. Heamostas 1986, 56, 407-410; see also 1.6.2 below) which uses a monoclonal antibody which does not recognize or bind the protein region including the GFD (see above for some specific examples of these antibodies) in parallel or serially with an immunoamidolytic assay with a monoclonal antibody which does recognize or bind to the protein region (such as MAB $CD_6$ or $DC_1$, see below for further details).

Monoclonal antibodies with this specificity may be prepared according to the known cells fusion techniques by applying a proper selection method. The immunizing agent may be scuPA, tcu-PA, ATF or a mixture thereof and usual immunization schedules can be followed. Antigen recipients may be any of the experiment animals used in this technique and preferably rodents such as rats, mice or rabbits. The stabilized cell line to be fused with the spleen cells from the treated animals is any of the known and currently available cell lines, such as preferably tumor cell lines, e.g. mouse myeloma NSO (subline of P3/NS1/ 1 Ag-4.1), ATCC TIB 18). The preferred probe for the selection of the MABs with the required features is a peptidic fragment (named $F_6$) having an apparent molecular weight of about 6000, an aminoacid sequence corresponding to the uPA sequence spanning approximately from the leucine residue in position 4 to the glutamic acid residue in position 43, and thus corresponding (being a relevant portion of or including) to the so-called GFD of uPA.

This fragment is obtained by controlled treatment of the ATF with an enzyme capable of cutting a protein sequence close to a residue of glutamic acid, such as <u>Staphylococcus aureus</u> derived protease V8. As positive controls in this selection system, it is sometimes convenient to introduce a further fragment obtained from ATF with V8 treatment, i.e. a fragment named $B_{11}$ having an apparent molecular weight of about 11000 and a sequence essentially corresponding to the uPA "Kringle" region, and optionally a third fragment named $F_{12}$ obtained by the same procedure having a sequence essentially corresponding to the uPA "Kringle" region but for a clipping between aminoacid 52 and 53.

The MABs that bind to fragment $F_6$ as well as to uPA (scuPA and/or tcuPA) without binding to the "Kringle" region (fragment $B_{11}$) or catalytic region (B-chain), e.g. in an immunoaffinity or immunoblotting experiment, are further purified, e.g. by affinity or ion exchange chromatography and isolated. They possess the desired feature of recognizing or binding selectively to the uPA GFD region.

To demonstrate that the GASGAs derivatives of the invention do not bind to the uPA receptor, such as that on U 937 monocytes described by Stoppelli MP et al in Proc. Natl. Acad. Sci, USA, 1985, 82, 4939-4943, an assay can be performed involving repeating the experiment described in this article but using the GASGA derivative of the invention instead of uPA, that may be introduced in the experiment only as a positive control. In this assay, the binding capacity of the GASGAs derivatives of the EO series is quite low. In general, it is lower than 10% the uPA binding capacity.

Instead of or in addition to, using U 937 monocytes the above experiment can be performed by using the human umbilical vein endotelial cells described by Ajjar KA et al. in J. Biol. Chem. 1986, 261, 11656-11662.

The in vivo activity of the compounds of the invention can be confirmed by experiments on animal models such as the clot lysis in anesthetized rabbits as described by Collen D. et al. in J. Clin. Invest. 1983, 73, 368-376.

The GASGAs derivatives of the invention can therefore be used as possible substitutes for scu-PA or uPA in the therapeutic applications so far devised for these substances, e.g. as thrombolytics. To this end, the compounds of the invention can be used as such or preferably formulated according to known per se techniques which are described in reference books such as Remington's Pharmaceutical Sciences, 17th Edition, 1985, Mack Publishing Co. In particular, they can be formulated analogously to the current formulation of uPA or scu-PA.

A preferred therapeutic indication is therefore as thrombolytics, possibly in the early stages of infarction or embolism, such as is heart infarction, pulmonary embolism or peripheral arterial embolism. The route of administration is preferably i.v., and the mode of administration can be in bolus or by perfusion. Preferably, an in i.v. bolus administration of a thrombolytically effective amount of a compound of the invention is followed, if necessary, by i.v. perfusion of a lower dosage for a relatively short period of time. The daily dosage vary greatly with the patient age, size, condition and can be indicatively in the range of 2-30 mg/die, preferably 2-20 mg/die and most preferably 2-10 mg/die. As known in the art, in this particular therapeutic field the appropriate dosage and administration scheme can be decided by the physician on a case by case basis.

The dosage unit, which contains the usual stabilizing agents as described for uPA, scu-PA or t-PA, can contain GASGAs in the range of 0.5-2 mg per unit, corresponding to about 50,000-200,000 IU of uPA per unit.

An example of a lyophilized pharmaceutical formulation for reconstitution in 2 ml sterile saline for e.v. injection is the following:

GASGA-E0 (lyophilized)     1 mg
mannitol     20mg
Sodium edetate     2 mg
phosphate buffer q.s.

The compounds of the invention can be administered alone, or in combined therapy with anticoagulant agents such as heparin or aspirin or with any of the other PA, such as scu-PA, uPA, t-PA, or modified forms thereof, such as those described in EP-A 231883 and EP-A 236040 streptokinase or mixtures thereof.

## Brief description of the drawings.

The accompanying drawings are intended to further illustrate the invention and the reference to the drawings in the description and claims is mainly by way of illustration and is not intended to be a limitation to the scope of the present invention. The following brief description of the drawings is intended to make the understanding of the drawings and of the invention as a whole easier.

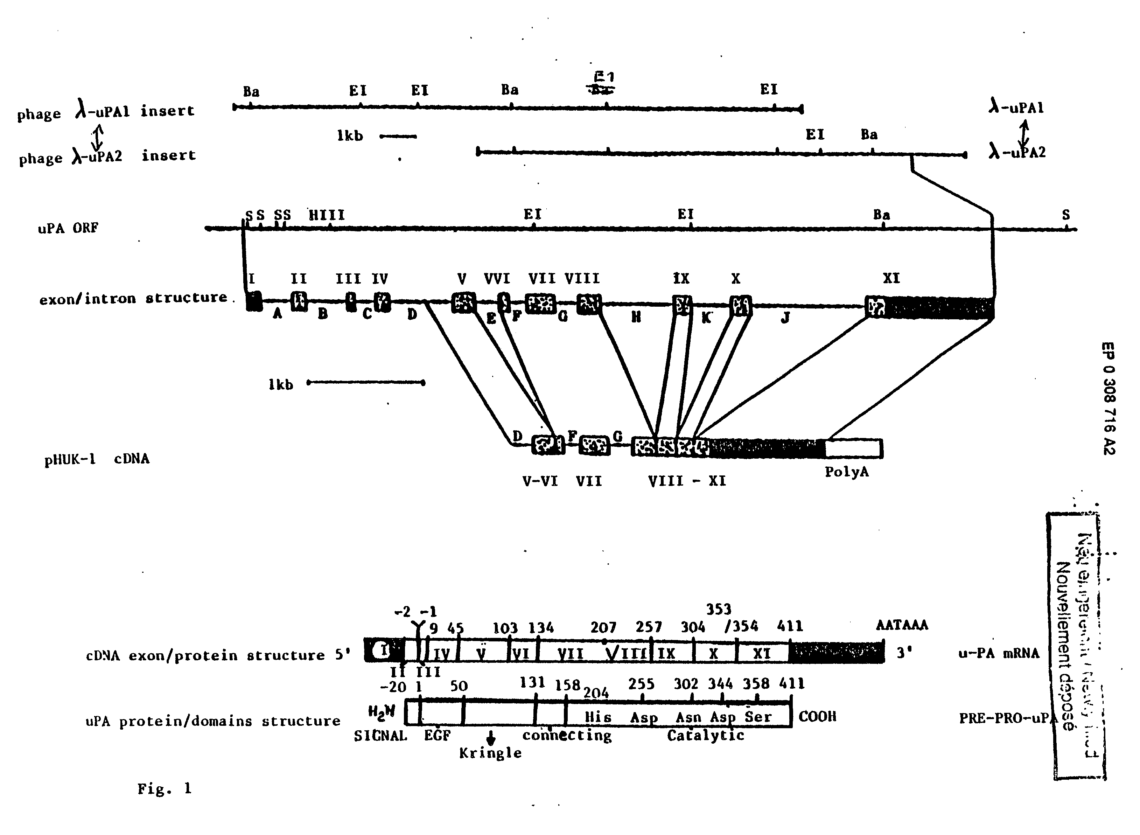

## Fig.1 Structure of the uPA gene, mRNA and protein

From the top to the bottom:
- restriction map of the uPA gene insert in phage lambda-uPA2;
- restriction map of the uPA gene insert in phage lambda-uPA1;
- restriction map of the uPA open reading frame (Ba: BamHI; EI: EcoRI; S: SmaI; HIII: HindIII);
- exon/intron structure of the human uPA gene: black boxes: untranslated 5′ and 3′ regions; shadowed boxes: translated exons; lines: introns;
- exon/intron structure of the partially unspliced cDNA clone pHUK1;
- correspondence between exons and aminoacids in the pro-upA mRNA;
- correspondence between protein domains and amino acids in pro-upA.

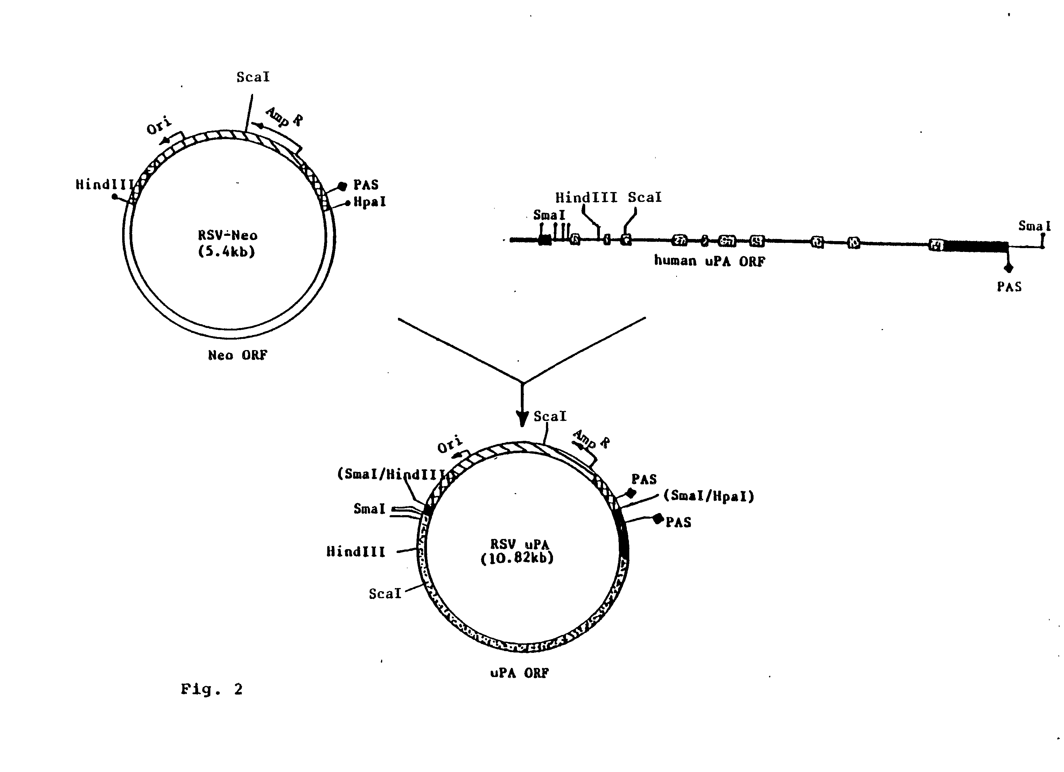

Fig.2 Costruction of RSV-uPA

A SmaI DNA fragment containing the complete ORF for the uPA mRNA obtained from lambda-uPA1 (see the top portion of this figure) is inserted in the frame obtained from the plasmid RSV-Neo by HindIII and HpaI digestion which carries the bacterial plasmid functions and the LTR of the RSV (see the left portion of this Figure). RSV-uPA has the uPA ORF under the control of the promoter contained in the RSV LTR and can express the protein when inserted in a suitable eukaryotic host (see the bottom portion of this Figure).

Fig.3 - Costruction of GASGAs expression vectors

Detailed restriction map and Exon/Intron structure of:
A) human uPA ORF (boxes: exons; lines: introns);
B) enlargement of the region surrounding ExonIV in plasmid RSVuPA showing also the nucleotides in codons which are splitted in two adjacent exons as well as the corresponding aminoacids;
C) same region as described above (Fig. 3)B, in $RSVuPA_{P>S}$ (left) and $RSVuPA_{B>X}$ (right);
D) structure of the GFD region in the ORF of RSVuPA-EO (left) and RSVuPA-E2 (right); the new codons and amino acids created in the chimeric plasmids are shown below the maps; the exons coming from $RSVuPA_{P>S}$ are identified as "1", while those from $RSVuPA_{B>X}$ are identified as "2".

Fig.4 Restriction map of pBPV-230.8

pBR322 derivative pML2dx is inserted at the unique BamHI site of the BPV-1 genome (Saver et al. Mol. Cell. Biol. 5, 3507; 1985) and the BamHI site adjacent to the 3' end of the transforming region is modified to SalI.

Fig.5 Construction of a human uPA expression cassette

Plasmid RSVuPA has two unique restriction sites, NdeI and APaI that are modified by XhoI linkering obtaining the plasmid p71. From the latter an uPA expression cassette (see the bottom of this figure) can be obtained by XhoI digestion. This fragment, which is suitable for insertion between two XhoI and/or SalI sites, can drive the trascription of the human uPA gene.

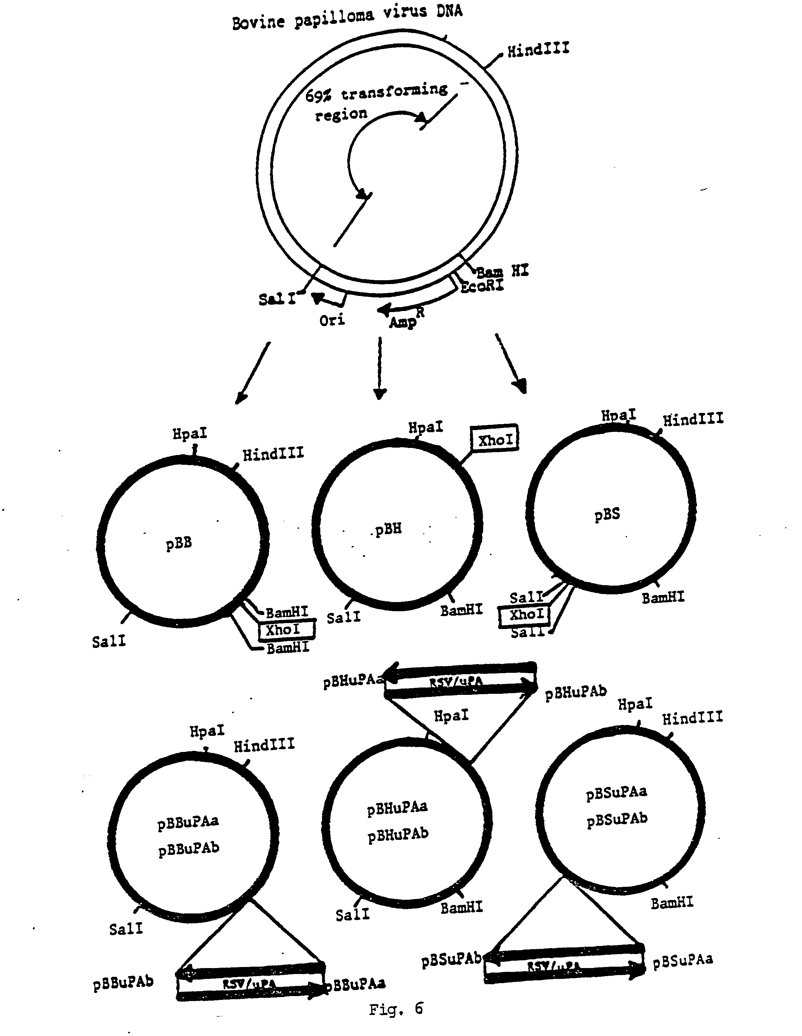

Fig.6 Insertion of the RSV-uPA construct in episomal vectors

Plasmid pBPV230.8 is modified by replacing one of the three viral sites BamHI, HindIII and SalI obtaining respectively pBB, pBH and pBS. The new XhoI site is boxed in each derived vector. The new BamHI and SalI sites resulting from the insertion of the XhoI linker and flanking the XhoI site in pBB and pBS are showed.
In the three plasmids the RSV/uPA construct (see Fig. 3) is inserted in the XhoI site in both orientations obtaining the following plasmids:
- pBBuPAa, pBHuPAa and pBSuPAa wherein uPA and BPV mRNAs are transcribed in the same sense;
- pBBuPAb, pBHuPAb and pBSuPAb, wherein uPA mRNA and the BPV mRNAs are transcribed in opposite direction.

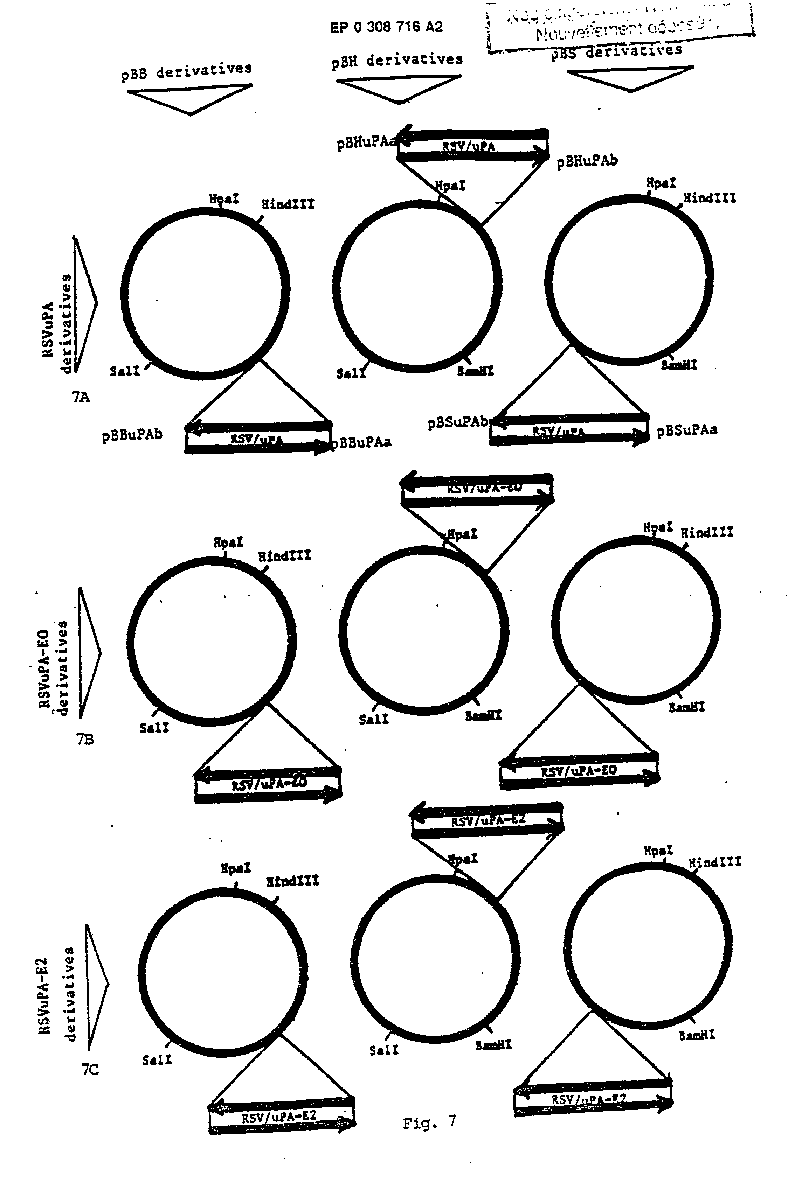

Fig. 7 - Modification of the uPA ORF in BPV derived vectors

Flow-chart of BVP derived vectors expressing uPA (A), GASGA-E0 (B) and GASGA-E2 (C). On the top are indicated the pBPV230.8 XhoI derivatives, on the left the RSV-Neo-derived expression vectors that donate the RSV/uPA cassette (uPA expression vectors) (Fig.7A) or the BssHI/SacI fragment (GASGAs expression vectors, Fig.7B and C) which derive respectively from RSVuPA-E0 and RSVuPA-E2.

9

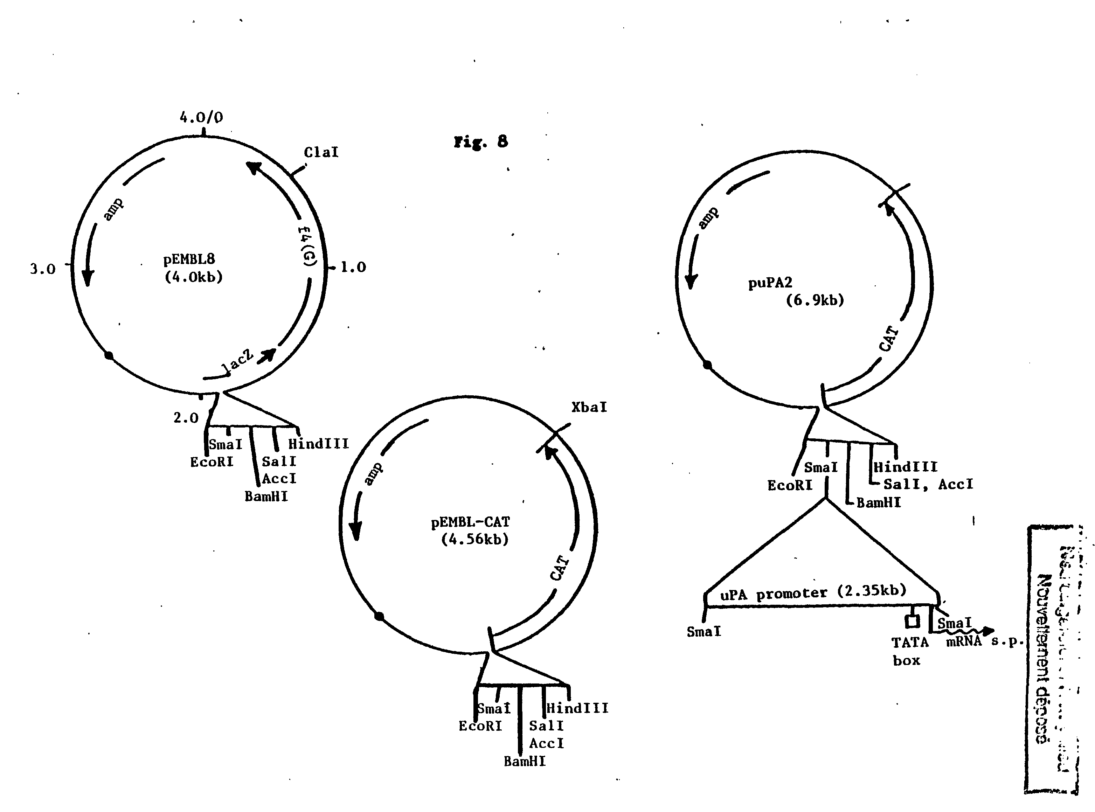

Fig.8 Construction of puPA2

Plasmid pEMBL8-CAT is derived from pEMBL8 by substituting the fragment between ClaI and HindIII with a fragment carrying the bacterial CAT gene (Dente et al., Nucl. Acid Res., 1645-1655; 1983). A SmaI fragment containing the uPA promoter is inserted upstream the CAT gene generating the plasmid puPA2 (this figure, on the right) which has the promoter in the proper orientation frame.

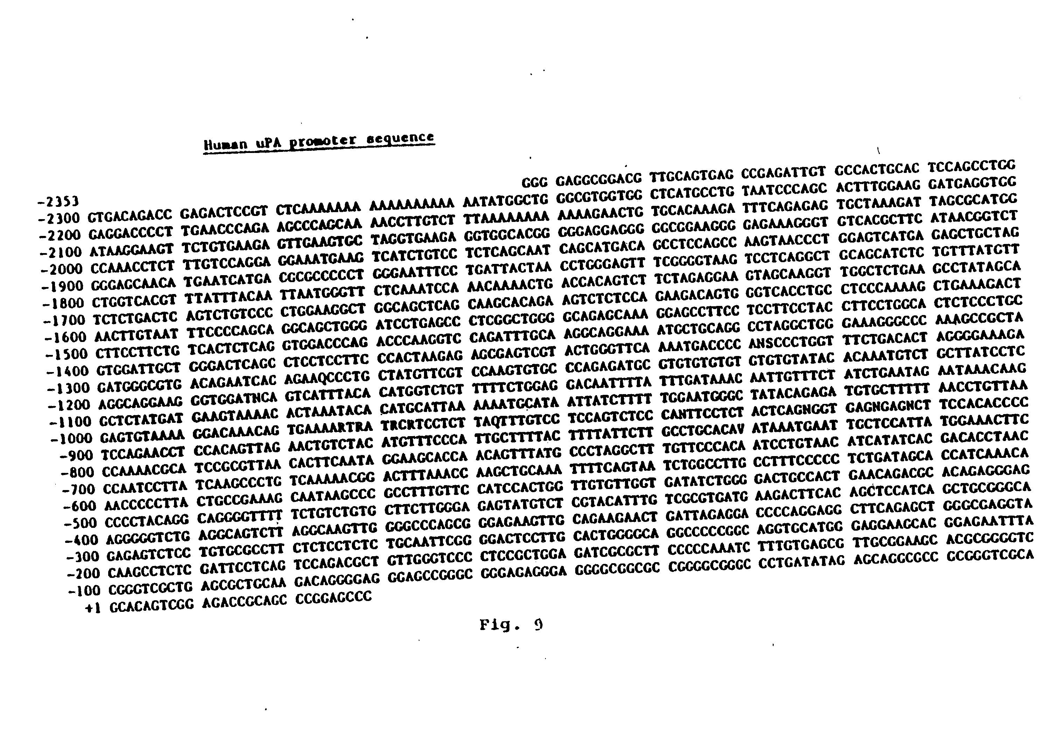

Fig.9 - Sequence of the human uPA promoter

The sequence of a 2.38kb SmaI fragment from lambda-uPA1 phage DNA, containing the mRNA start point, the TATA box and the upstream regulatory regions of the uPA promoter, is shown. Nucleotide +1 on the map corresponds to the first nucleotide in the uPA mRNA described by Riccio et al. using primer extension analysis.

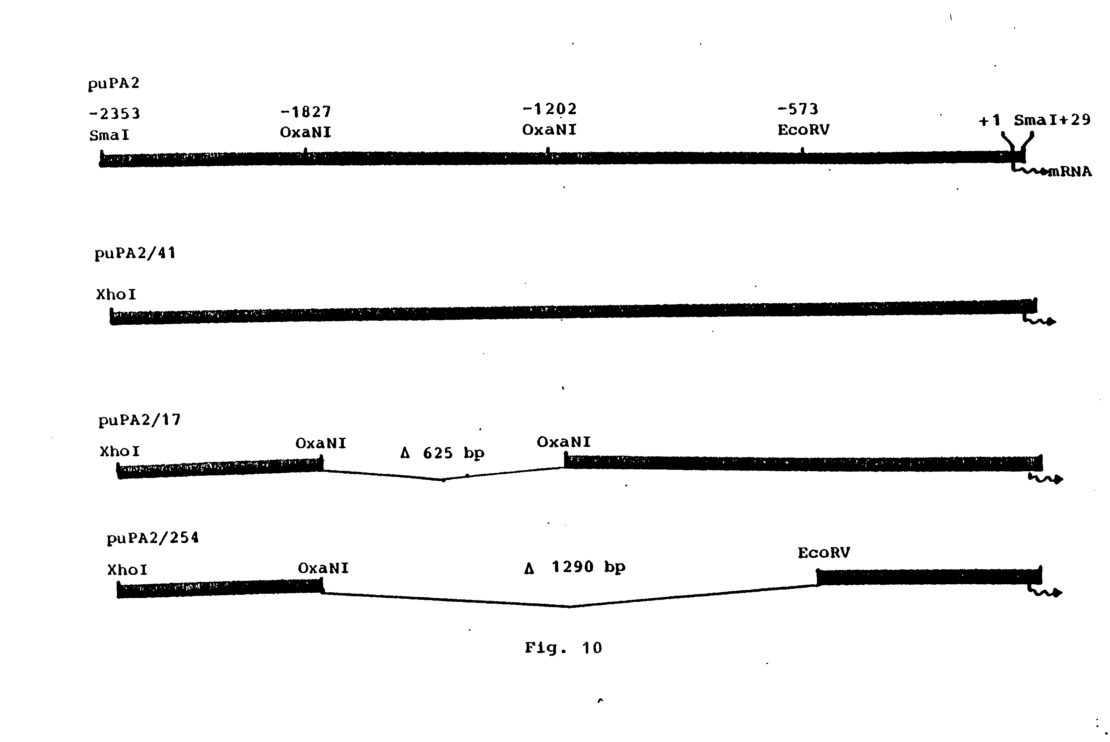

Fig. 10 - Restriction map of the uPA promoter and its derivatives

The restriction map of the puPA2 modified plasmids indicating the regions of where the uPA promoter has been modified is represented; in particular:
- puPA2/41, wherein the SmaI site at the 5' end of the promoter is converted to XhoI;
- puPA2/17, wherein the region between the two OxaNI sites has been deleted;
- puPA2/254, wherein the region between the 5' OxaNI site and the EcoRV site has been deleted.

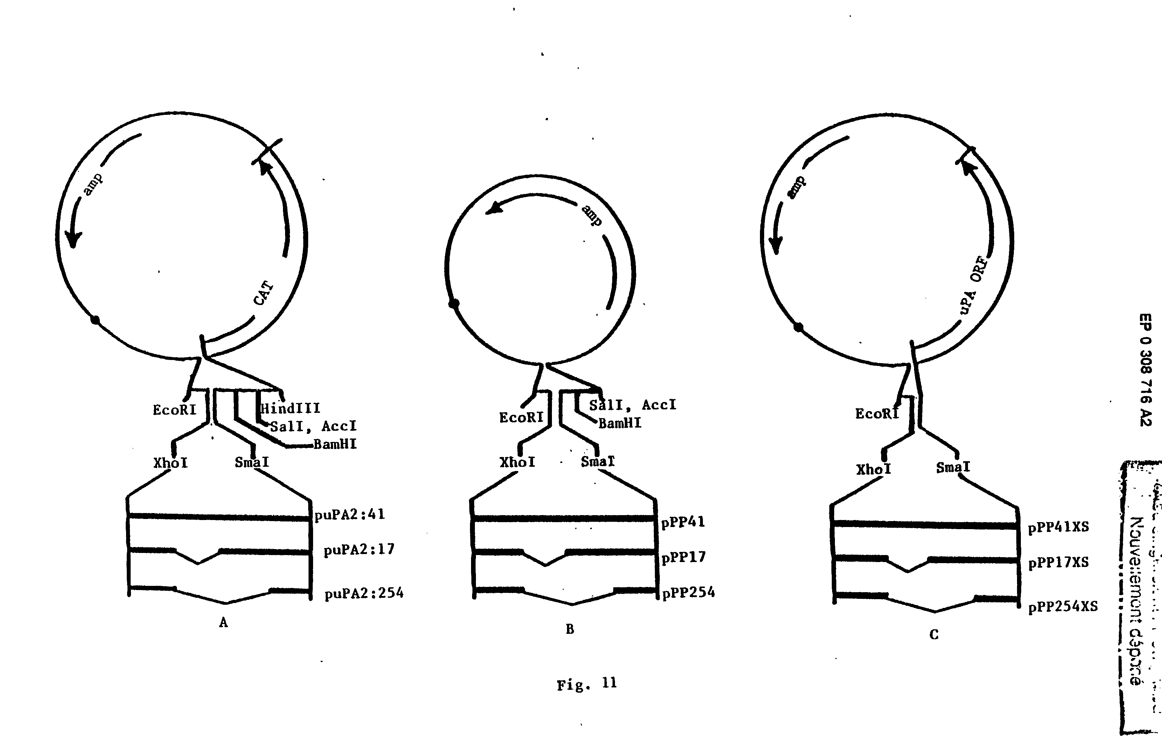

Fig. 11 - uPA promoter derived vectors

Fig. 11A depicts the restriction map of plasmids puPA2/41, puPA2/17 and puPA2/254
Fig. 11B pPP17 and pPP254 that are obtained from puPA2/41, puPA2/17 and puPA/254, respectively, by excising the CAT gene by SalI/XbaI digestion and SalI linkering. These vectors contain the uPA promoter (or a derivative thereof), the TATA box, and the mRNA start point and can be used to express genes or cDNAs by inserting them, for example, at the blunt-ended SmaI site or at the BamHI site with fragments having MboI, XhoII, BglII, BclI and BamHI ends. These constructs can be excised by double digesting with XhoI and SalI. The same double digestion can be used to excise the promoter construct to insert it, as an "expression cassette", in a different plasmid 5' to the gene or cDNA to be expressed.
The filled circle indicates the plasmid origin of replication.
Fig. 11C depicts plasmids pPP41XS, pPP17XS, and pPP254XS, which are derived from pPP41, pPP17 and pPP254, respectively, by inserting a 7.3 kb SmaI fragment containing the uPA ORF in the plasmid SmaI site.

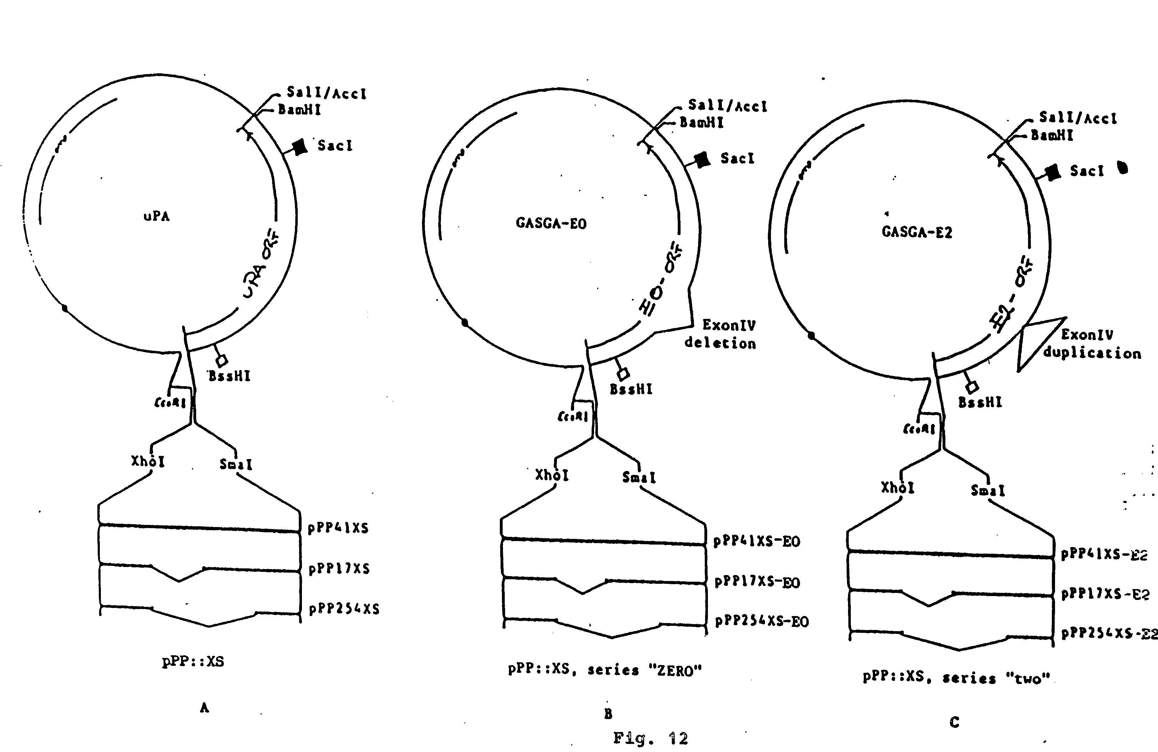

Fig.12 - Costruction of minigenes derived GASGAs expression vectors

uPA expressing vectors (pPP::XS, Fig. 12A) are modified in the uPA ORF to delete Exon IV (pPP::XS-E0, Fig. 12B) or to duplicate it (pPP::XS-E2, Fig. 12C).

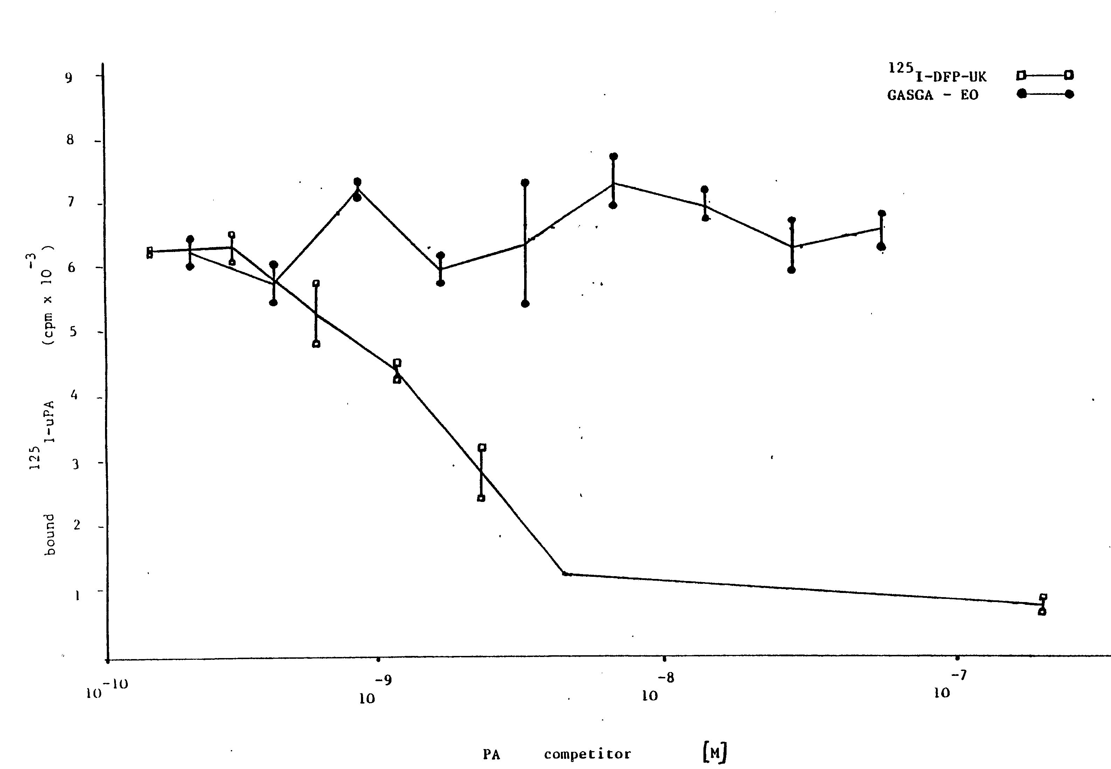

Fig.13 - uPA receptor binding essay

The figure shows the inhibition of the binding of $^{125}$I-DFP-UK to the uPA receptor on U937 cells when culture supernatants from CHO cells transformed with GASGA-E0 vector are added. Unlabelled uPA is present as the positive control.

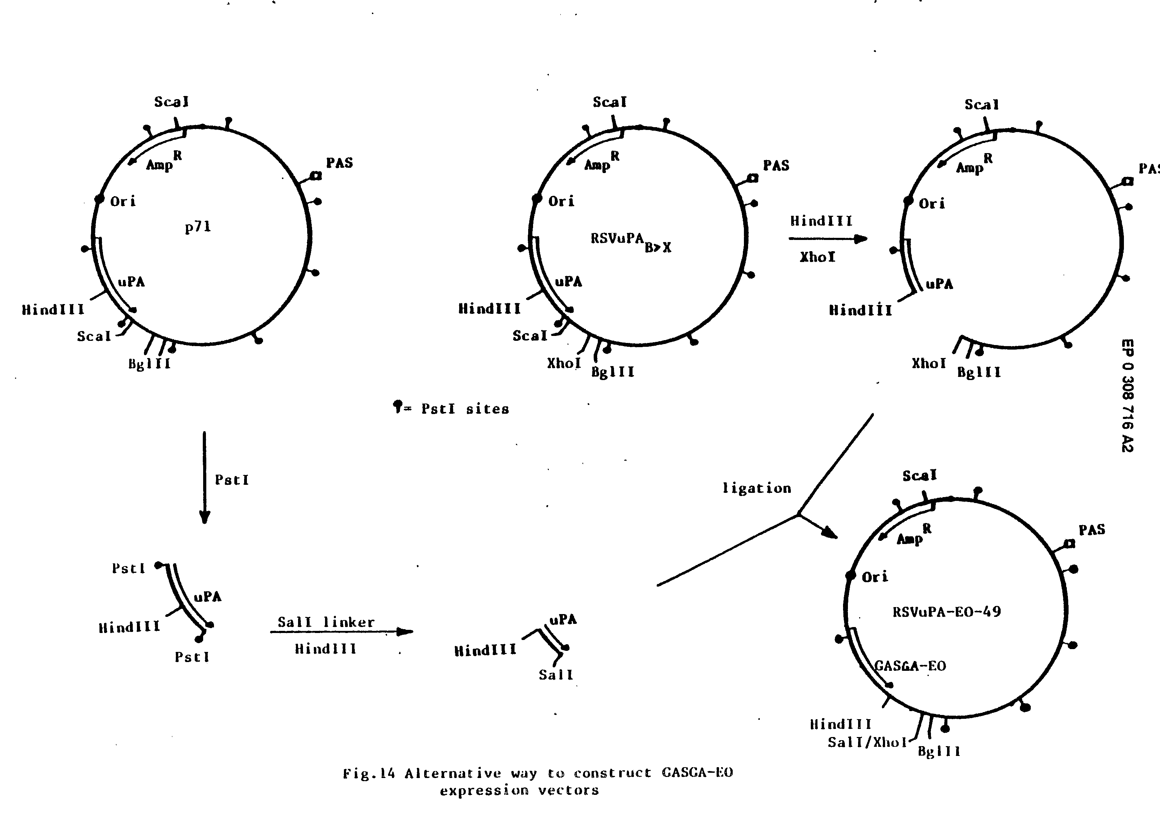

### Fig.14 - Costruction of RSVuPA-EO-49

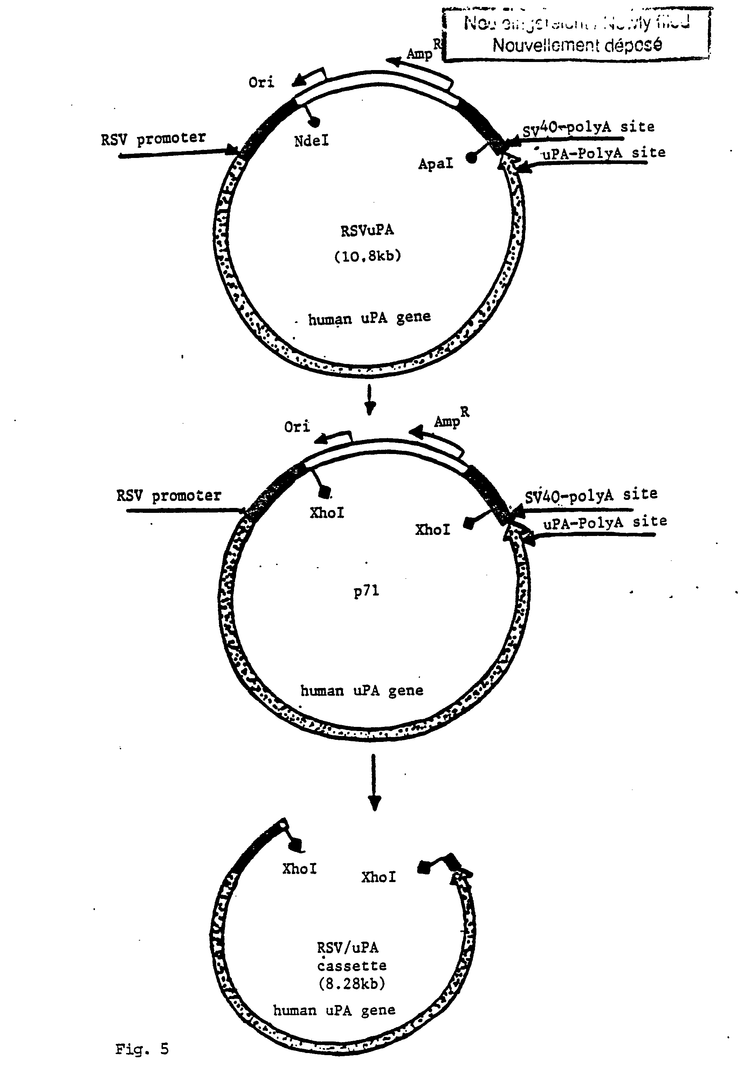

RSV-uPA-EO-49 is an expression vector containing the uPA ORF with a deletion substantially corresponding to exon IV (i.e. the GFD coding region) (c.f. this figure, right bottom portion). It is obtained by ligating a RSV-uPA$_{B>x}$ fragment containing most of the plasmid but lacking exons III and IV (this figure, upper right portion) with a HindIII-SalI plasmid fragment spanning from the 3' portion of intron B to the 5' portion of intron C (see the bottom of this figure, in the middle). The RSV-UPA$_{B>x}$ fragment mentioned above is obtained by HindIII and XhoI digestion of RSV-uPA$_{B>x}$ (fig. 2), while the HindIII-SalI fragment (which substantially corresponds only to exon III) is obtained by HindIII digestion of a plasmid fragment derived from PstI digestion of p71 (see fig. 5) whose ends have been modified by SalI linker.

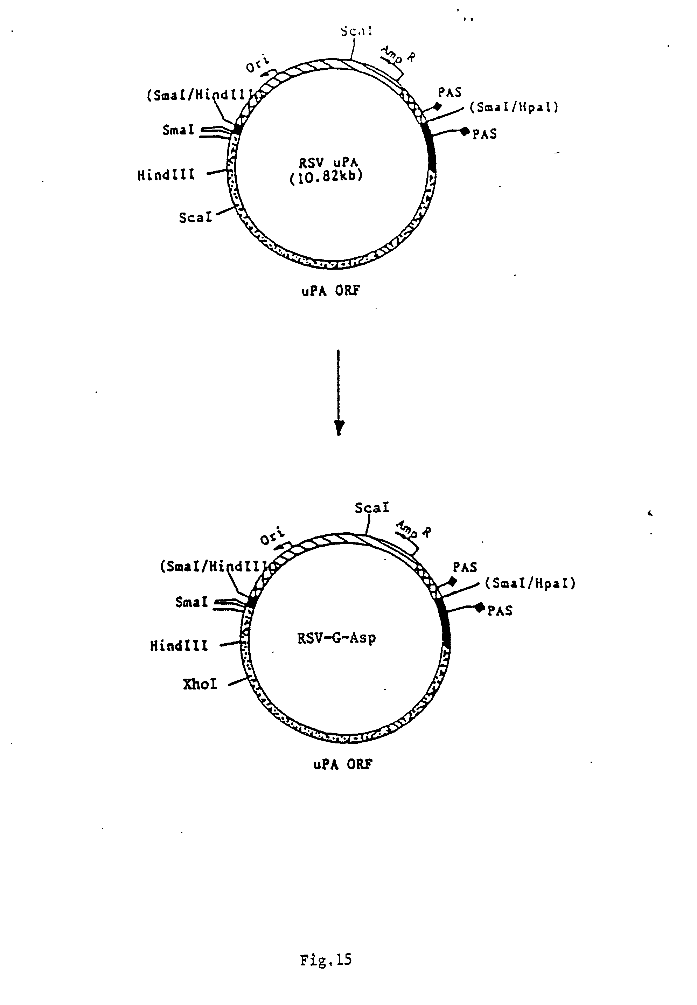

### Fig.15 - Costruction of RSV-G-Asp

Plasmid RSV-G-Asp (see the bottom portion of this figure) is constructed by partially digesting RSVuPA (see the upper portion of this figure) with ScaI, modifying the ends with a Asp718 linker and selecting for the appropriate restriction pattern. This vector has the uPA ORF downstream from the insertion which is out of frame.

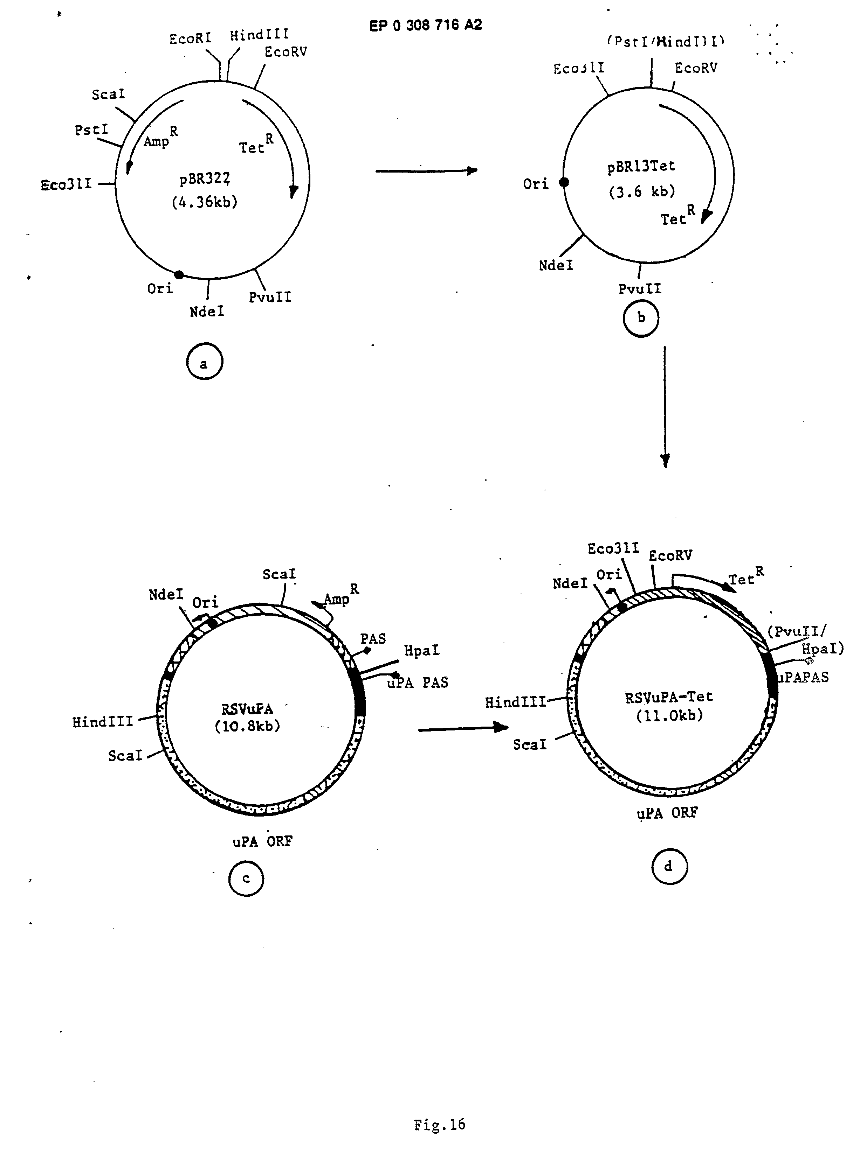

### Fig.16 - Costruction of RSVuPA-Tet

Plasmid pBR13Tet is represented in the bottom portion of this figure (d). It is obtained by deleting most of the ampicillin resistence gene in pBR322 by digesting with PstI and HindIII and recirculatizating the plasmid after having blunted the protruding ends.

From this plasmid (pBR13Tet see b) a NdeI/PvuII fragment containing the Tetracycline resistence gene and the origin of replication has been ligated to NdeI/HpaI fragment from RSVuPA (see c) containing the RSV promoter and the human uPA gene in addition to its own polyadenilation site (PAS).

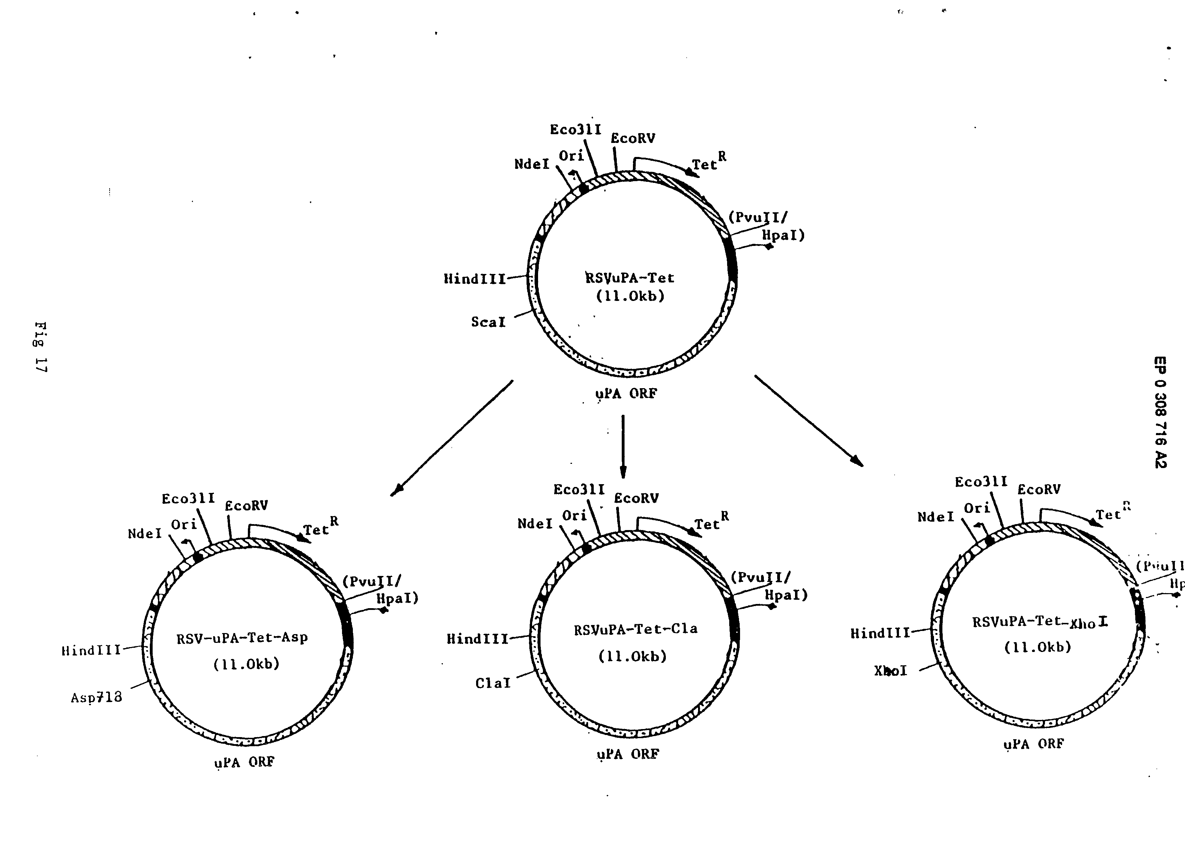

### Fig.17 - Costruction of linker modified RSVuPA-Tet derivatives

Plasmid RSVuPA-Tet (see the upper portion of this figure), containing a unique ScaI site in the uPA exon IV, is linearized with ScaI and modified by linkering with an Asp718 linker obtaining the plasmid named RSVuPA-Tet-Asp (bottom left), a ClaI linker obtaining a plasmid named RSVuPA-Tet-Cla (bottom middle) or a XhoI linker a plasmid named RSVuPA-Tet-XhoI (bottom right). These plasmids have the uPA coding region downstream from the insertion which is no longer in frame.

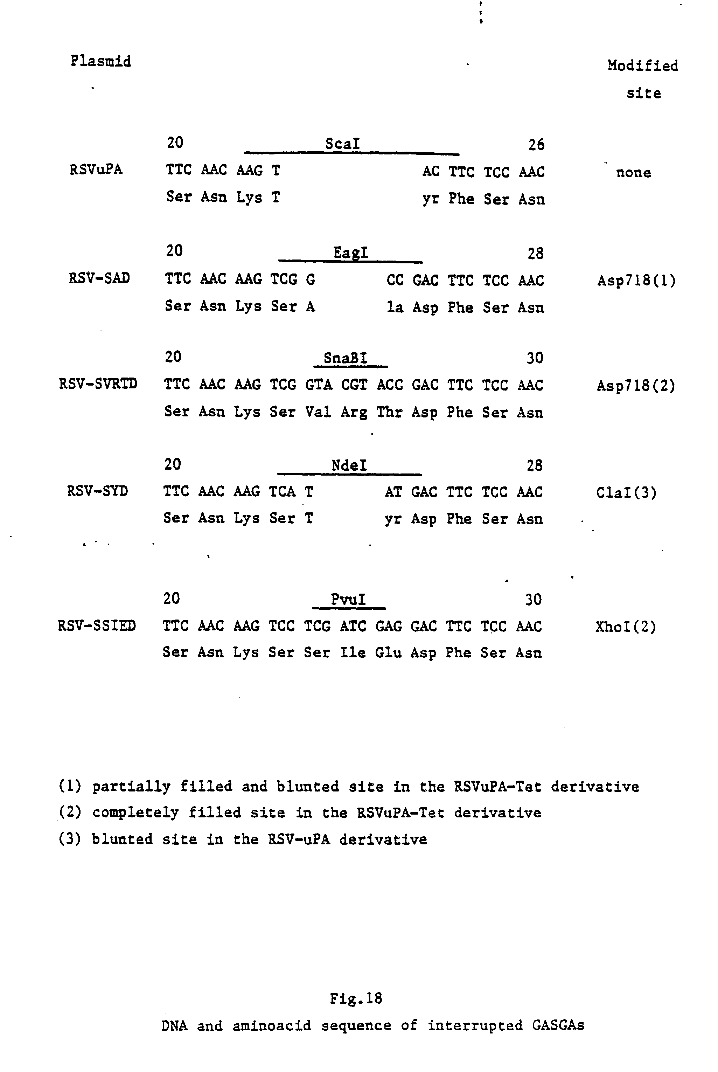

### Fig.18 - DNA and aminoacid sequence of interrupted GASGAs

Plasmids RSV-uPA-Tet-Asp, RSV-uPA-Tet-Cla, RSV-uPA-Tet-XhoI which have the uPA coding region downstream from the insertion out of frame are separately modified by digestion with the enzyme specific for the polylinker derived site and partial or full filling of the protruding ends to reinstate the proper reading frame. The DNA and aminoacid sequence close to the modification are reported in this figure.

The column headed "modified site" refers to the modified site of the starting plasmid (e.g. Asp 718 indicates the unique site of RSV-uPA-Tet-Asp which is used as the starting material to obtain the modified plasmid reported in this figure, namely RSV-SAD in the case of partial filling or RSV-SVRTD in the case of complete filling). This figure shows also the restriction sites introduced with the filling.

## EXAMPLES

The following examples are intended only as an illustration of the invention and of the way in which it can be exploited, but cannot be construed as a limitation upon its scope. To make the understanding of the following examples easier, we report in the paragraphs preceeding the examples (from 1.1. to 1.6.5) the techniques and methods (such as DNA enzymatic modification, DNA precipitation, protein purification, etc.) which are applied, essentially in the same manner, in different examples. In the examples, in fact, instead of repeating the whole procedure, the paragraph where it is described is oftentimes referred to.

1.1. The cell used are commercially available or otherwise available through the scientific community or authorized repository.

1.2. The cells are grown in an appropriate medium such as Dulbecco's modified Eagle medium (DMEM) containing 10% heat-inactivated fetal calf serum.

1.3 - Media, buffers and solutions for molecular biology.

When available, reagents are "DNA/RNA grade" and "analytical grade" in all the other cases. If not differently indicated, liquid and solid media for bacteria and phage growth, buffers and solutions are prepared, stored and used as as indicated in Maniatis T., Fritsch E.F., Sambrook J., Molecular Cloning, a laboratory manual, Cold Spring Harbor Laboratory, (1982), herein "Maniatis et al". If not differently indicated, glassware, plasticware and laboratory hardware are prepared, stored and used as indicated in the same reference. Deionized/distelled water is used, autoclaved if to be used in enzyme buffers.

1.4 - Enzymes for molecular biology.

Restriction endonuclease, DNA modyfing enzymes and RNase are commercially available. We used those purchased from Boehringer, Bethesda Research Laboratories (BRL), Pharmacia; lysozyme and bovine serum albumine (BSA) are also commercially available. We purchased lysozyme from Sigma and BSA from Boehringer. Commonly used buffer solutions are used for each enzyme reaction. Their composition and preparation are generally known and conveniently they are prepared according to the information accompanying each enzyme, the preparations are filter sterilized and stored in aliquotes at -20° C. Many buffers of current use are also commercially available as concentrates. Synthetic DNA linkers are commonly available as well as t-RNA and herring sperm DNA. We purchased synthetic DNA linkers from P-L Pharmacia or New England Biolabs, while tRNA and herring-sperm-DNA were from Boehringer.

1.5 - rDNA tecniques.

If not differently indicated in the following examples, rDNA techniques are performed as described in Maniatis et al. and in the original references therein contained.

1.5.1 - Restriction digestions and DNA enzymatic modification.

Single and multiple restriction digestions and DNA enzymatic modifications are performed as known in the art, and also reported in commonly available manufacturers' booklets and in Maniatis et al, ch.4. "Heat inactivation" means inactivating the enzyme at 70° C for 10-15 min, then leaving the mixture from 10 to 15 min at room temperature and spinning down 2 min in an Eppendorf centrifuge. Preferentially, restriction digestions and the results of enzymatic DNA modification are checked on 0.5 microg/ml ethidium bromide containing 1% agarose gels runned at 100 V for 1-2 h (see also Maniatis et al., ch.5).

1.5.2 - Phenol/chloroform extractions.

Preferentially, phenol/chloroform extractions of proteins from nucleic acid aqueous solutions are performed as described in Maniatis et al., p. 458; DNA is "back-extracted" when the reaction volume is smaller then 200 microl.

### 1.5.3 - DNA precipitation.

Preferentially, DNA precipitations are performed adding 0.1 volumes of 3M sodium acetate pH 5.2 and 3 volumes of ethanol (at -20° C). 20 to 40 microg of tRNA are added only when indicated in the following examples. The mixture is left in a dry ice/ethanol bath for 15 to 30 minutes or at -20° C overnight and then spinned down at 4° C for 15 min at 10000 x G in an Eppendorf microcentrifuge or in a Sorvall centrifuge. The DNA pellet is washed once with 70% ethanol and then once with 100% ethanol and finally dried in a Savant vacuum centrifuge.

### 1.5.4 - 5' Dephosphorilation.

Preferentially, 5' DNA ends are dephosphorilated with commercially available calf intestine phosphatase (CIP),(from Boehringer), at 37° C for 1$^h$. CIP is removed by phenol:chloroform extraction, or by adding EGTA ethylene bis (oxyethylenenitrilo) - tetracetic acid to a final concentation of about 50mM and heating at 68° C for 45 min.

### 1.5.5 - DNA ligation.

DNA ligations are performed as known in the art. Conveniently, T4 DNA ligase manufacturers' instructions are followed. Conveniently, Ligase reactions are performed at 8° C when synthetic linkers are used, at 14 to 16° C in all the other cases. When blunt-ended DNA is to be ligated, PEG 1500 or PEG 4000 is added to a 10% final concentration. Conveniently, 10 x ligation buffer of the following composition is used: 0.66 M Tris-HCl pH 7.6, 50mM $MgCl_2$, 50 mM dithiothreitol, and 5mM ATP.

### 1.5.6 - DNA linkers.

Preferentially, linker DNAs are used, phosphorylated and checked as indicated in Maniatis et al., pages 125-126, and used as indicated at page 392 and seq. Kinase reaction, ligation and restriction of the linker alone and of the linkered DNA fragments are checked by electrophoresing the linker on a 2% agarose gel for 10 to 20 min and for 1 to 2 hours in case of the linkered fragments, and then visualizing the DNA by autoradiography as indicated in Maniatis et al., pag.172.

### 1.5.7 - Preparative gel electrophoresis.

Preferentially, preparative gel electrophoresis is performed on 0.7% to 0.8% agarose gel containing 0.5 microg/ml ethidium bromide in 1X TBE buffer (0.089 M Tris, 0.089 M boric acid, and 0.002 M EDTA, Maniatis et al. pag. 156). The DNA is runned at 25V for the first 2 h and then at 50V for 16 to 20 h. DNA fragments are visualized on a 305nm UV transilluminator and DNA bands are cut out and electroeluted in an electroelution system (ISCO) in 0.1X TBE (see above), at 400V for 10 to 15 min. DNA is then purified on Elutip-D column (Schleicher & Schuell) prepared and rinsed with a low salt buffer and eluted with a high salt buffer. Typically the column is prepared in a buffer containing 0.2 M NaCl, 20 mM TRIS-HCl pH 7.3-7.5 (TRIS is 2-amino-2-hydroxymethyl-1,3-propanediol) and 1.0 mM EDTA. This buffer is also used for rinsing, while the elution buffer contains 1.0 M NaCl, 20 mM TRIS-HCl pH 7.3-7.5, and 1.0 mM EDTA. Finally the DNA is precipitated as indicated in 1.5.3.

### 1.5.8 - E. coli transformation.

Preferentially, E.coli transformation (c.f. Hanahan D., Studies on transformation of E. coli with plasmides, J. Mol. Biol. (1983) 155 557-580) are performed using commercially available or otherwise available competent cells such as strains DH1, DH5 or HB101 of BRL (Bethesda Research Labs) according to known per se techniques which are summarized also in the manufactures' booklet. Conveniently, the 100 microl

13

(BRL) protocol is used when a DNA fragment is to be inserted in a different plasmid, while the small-scale, 20 microl (BRL) protocol is used when converting restriction sites by DNA linkers. After incubation without antibiotic, the cells are plated on agar LB plates (Maniatis et al. p.440-444) and incubated overnight at 37° C in the presence of the appropriate antibiotic.

1.5.9 - Screening of the transformed cells (miniprep analysis).

Preferentially, resistant colonies from DNA transformed competent E.coli cells are screened by growing isolated colonies overnight at 37° C in 5ml of LB and the appropriate antibiotic (Maniatis et al., p. 440) and analyzing samples (1.5 ml) of the liquid culture with the boiling method as reported in Maniatis et al., p.366-367. Conveniently the following variations may be introduced:
- reaction volumes are scaled up 1.5 times;
- E.coli strains DH1 and DH5 are boiled for 60 sec;
- DNA precipitation is carried out without adding salt, but adding 1 vol of isopropanol and leaving the tubes at -20° C for 20 to 25 minutes;
- precipitated DNA is washed once with 1ml 70% ethanol and once with 1ml 100% ethanol;
- DNase-free RNase is added at 10 microg/ml and the DNA incubated 20 min at 70° C.

1.5.10 - Plasmid large scale preparation.

Preferentially, plasmid large scale preparations are performed as described in Maniatis et al., ch.3. Preferentially the alkaline lysis method is used to recover the plasmid DNA (c.f. Maniatis et al, p.90-91), the plasmid DNA is purified twice on ethidium bromide containing CsCl gradients, then the mixture is extracted with isoamylic alchool, and the aqueous DNA containing phase is separated and dialized against TE pH8 - (Maniatis et al.) and stored at 4° C.

1.6 - Purification and characterization of the obtained GASGAs

1.6.1 - Chemicals:

all the chemicals used are either broadly commercially available or easily preparable from commercially available products. For example, the following list includes some chemicals used in the following examples and their sources: Affi-Gel 10 (activated agarose), acrylamide, bisacrylamide, sodium dodecyl sulfate, Comassie Brilliant Blu were from Bio Rad, Richmond, USA; CNBr-activated Sepharose, electrophoresis calibration kit for low molecular weight proteins were from Pharmacia, Uppsala, Sweden. Ampholine were from LKB AB, Bromma, Sweden; bovin serum albumin (BSA), Chon fraction V, porcin trypsin, porcin plasmin, fibrinogen fraction I type IV, benzamidine, were from Sigma Chemical Co, St. Louis, MO 63178, USA; aprotinin was from Bayer, Leverkusen, GFR, glycopeptidase F from Boehringer Mannheim GmbH, FRG; S-2444 from Kabi AB, Stockolm, Sweden. High molecular weight urokinase (HMW-uPA) and the so called amino terminal fragment (ATF) were produced by Lepetit Research Laboratories SpA Milano, Italy. Scu-PA (pro-uPA) can be prepared substantially following the procedure described by Stump D.C., Thienpoint M., Collen D., J. Biol. CHem. 261, 1267-1273, 1986. A431 scu-upA is obtained according to the method described by Corti A., Nolli M.L., Soffientini A., Cassani G., in Thromb. Haemostas 1986; 56, 219-224. All other reagents were analytical grade products commercially available from Merck Darmstad, GFR or Carlo Erba, Italy.

1.6.2 - Immunological and activity assays:

The fibrinolytic assay is carried out by the fibrin plate method (Astrup T., Mullertz S., The fibrin plate method for estimating fibrinolytic activity, Arch Biochem Biophys 1952, 40:346-351). Urokinase reference standard is used to calibrate each assay. Activity is measured in International Units (I.U.) relative to the International Reference Preparation as the primary standard. sc-uPA (cell produced single chain urokinase) fibrinolytic latent activity is determined after activation as follows: plasmin (10 mg) is coupled with CNBr-activated Sepharose (cross-linked agarose) (2.5 ml) using 0.5 M sodium carbonate buffer as the coupling buffer. A 1:10 suspension of plasmin-agarose (0.9 ml) in 0.15 M sodium chloride, 0.05 M sodium phosphate buffer pH 7.4 is mixed with sc-uPA samples (0.1 ml) and gently rotated for 1 h at 20°C. Plasmin-agarose is removed by centrifugation 2 min at 1000 x g and the supernatant tested by the fibrin plate assay.

Protein concentrations were measured according to the method of Lowry (Lowry O., Rosebrough H., Parr A., Randall R., Protein measurement with the Folin phenol reagent, J. Biol. Chem. 1951, 193, 265-275). Amidolytic assays are performed by measuring the rate of hydrolysis of the synthetic substrate pyro-Glu-Gly-Arg-pNA, S-2444. (Claeson G., Friberger P., Knos M., Eriksson E., Methods for determination of pre-kallikrein in plasma, glandular kallikrein and urokinase, Haemostasis 1978; 7:76-78). Immunoadsorbent-amidolytic assay (IAA) is carried out as described by Corti A., Nolli ML., Cassani G., Differential detection of single-chain and two-chain urokinase type plasminogen activator by a new immunoadsorbent amidolytic assay (IAA), Thromb. Haemostas 1986, 56:407-410. The assay combines the selectivity of immunoassays with the specificity of enzyme activity assays exploiting both the antigenic and enzymatic properties of the two proteins.

In separated assays two types of monoclonal antibodies are used:

a) a monoclonal antibody recognizing the protein region which includes the GFD domain without recognizing either the catalytic portion of the protein or the Kringle (such as MAB $CD_6$ or $DC_1$)

b) a monoclonal antibody recognizing the protein catalytic domain without interfering with the enzymatic activity and without recognizing the region including the GF domain (such as those cited above in the description).

GASGAs bind to the monoclonal antibodies of point a) above, but do not bind to the MABs of point b), while on the contrary, scuPA/tcuPA bind to both series of MABs. GASGAs, ATF and scuPA/tcuPA bind to MABs recognizing the Kringle such as MAB 5B4.

## 1.6.3 - Reverse phase FPLC :

Reverse phase chromatography is carried out with a FPLC (Fast Protein Liquid Chromatography) system (Pharmacia Fine Chemicals) using a pro-RPC HR5/10 reverse phase chromatographic column (macroporous, microparticulated silica with bonded $C_1/C_8$ groups), Eluting buffers: buffer A, 0.1% trifluoroacetic acid in water, and buffer B, 0.1% trifluoroacetic acid in acetonitrile; elution mode: 100% A for 20 min, linear gradient from 0 to 60% B in A in 65 min, 100% B for 25 min; flow rate 0.3 ml/h.

## 1.6.4 - Electrophoretic procedures:

SDS-polyacrylamide gel electrophoresis (SDS-PAGE) is carried out according to Laemmli UK, (Cleavage of structural proteins during the assembly of the head of bacteriophage T4, Nature 1970, 227:680-685), in gel Slab. (convenienlty 1.5 x 120 x 150 mm). The stacking and separating gels contained 3% and 12.5% acrylamide respectively. Marker proteins (Ampholines) of the commercially available electrophoresis calibration kit for molecular weight determination (Pharmacia) are run in each gel. 2-Mercaptoethanol is used as the reducing agent and Comassie Brilliant Blue R250 is used for gel staining. When gel with a different acrilamide content is used, it is indicated generally between parenthesis. Immunoblotting is carried out essentially as known (c.f. Salerno G., Verde P., Nolli ML., Corti A., Szots H., Meo T., Johnson J., Bullok S., Cassani G., Blasi F., Monoclonal antibodies to human urokinase identify the single-chain pro-urokinase precursor, Proc Natl Acad Sci USA 1984, 81:110-114), using purified monoclonal antibodies which does not recognize or bind the region containing the uPA GFD such as those which recognize the uPA B-chain without recognizing the A-chain, which are described by Herion et al. in Bioscience Reports 1, 885-892 (1981), Salerno M.G. et al., Proc. Natl. Acad. Sci. USA, 1984, 81, 110-114, or Kaltoft et al, Proc. Natl. Acad. Sci. USA, 1982, 79, 3720-3723 or a monoclonal antibody binding to the A-chain only in the "Kringle" region such as MAB 5B4 (see above, Nolli et al.).

1.6.5. - Purification of GASGAs

GASGAs are purified from cell culture supernatants by immunochromatography and ion exchange FPLC as previously described for the purification of A431-uPA from A431 tumor cells supernatants (Corti A., Nolli M.L., Soffientini A., Cassani G. Purification and characterization of single-chain urokinase-type plasminogen activator (pro-urokinase) from human A431 cells, Throm Haemostas 1986; 56:219-224).

An agarose modified matrix bearing a monoclonal antibody which binds the uPA B-chain without binding the A-chain (such as MAB AAU2 described by Herion P. and Bollen A. in Bioscience Reports 3, 373-379, 1983) is equilibrated with 0.15 M sodium chloride, and 0.05 M sodium phosphate buffer pH 7.4 containing 1% polyethylenglycol (PEG) 6000. The column is loaded with 2 liters of cell supernatants at a flow rate of 50 ml/h, at 4°C, and then washed with the equilibrating buffer. Desorption is carried out with 1 M sodium chloride and 0.1 M acetic acid containing 1% PEG 6000. All fractions are tested by IAA (see 1.6.2) and those fractions which contain GASGAs are collected, pooled according to their content and further purified and concentrated by ion exchange chromatography in an FPLC (fast protein liquid chromatography) system (Pharmacia Fine Chemicals, Sweden) using a Mono S HR5/5 column (strong cation exchanger based on beaded hydrophilic resin with narrow particle size distribution; the charged group is $-CH_2SO_3H$) pre-equilibrated with 0.05 M sodium acetate buffer, pH 4. The sample is generally two-fold diluted with distilled water to reduce the ionic strength and then loaded on the column. After washing with the equilibrating buffer, the bound proteins are eluted with a salt/pH linear gradient mixture of buffer A (0.05 M sodium acetate buffer, pH 4) and buffer B (1 M sodium chloride, 0.05 sodium acetate, pH 5) from 30 to 70% B in A in 50 min, then 100% B for 20 min; flow rate, 60 ml/h. The cell supernatant content of enzymatically active GASGAs and enzymatically inactive, plasmin activable GASGAs antigen is measured by the immunoadsorbent-amidolytic assay (IAA) (see 1.6.2). The enzymatically activable GASGAs in the final product amounted to about 95% of the total. Reverse-phase FPLC analysis of GASGA resulted in a single peak of adsorbance at 280 nm.

Alternatively, the above procedure is carried out on an affinity matrix which is a Sepharose-MAB 5B4 matrix prepared as described by Corti et al (see above). The purification efficiency is similar to that reported above.

Example E0

Human Genomic Library

A library of human genomic DNA in bacteriophage lambda Charon 28 is prepared by techniques known in the art (c.f. P.H. Hieter et al., Cell, Vol. 22, 197-207 (1980) and Maniatis et al.). The recombinant lambda lysate is titrated on E. Coli K12 strain LE 392 (Maniatis et al. p. 504). Techniques for phage titration, as well as for screening of a phage library with a nick-translated probe, are also well known in the art (Maniatis et al.) and are applied substantially as such in the following experiments. For screening, 10 microliters of a one thousand fold dilution of the lysate are adsorbed onto 0.3 ml of a 2.5 fold concentrated culture of E. Coli K12 LE 392 in 10 mM $MgSO_4$ and incubated for 20 minutes at 37°C. 7 ml of 0.7% agarose is added and the whole mixture is layered onto 150 mm plastic plates containing NZYMC agar. (See Maniatis et al.). A total of 0.41 ml of a $10^{-3}$ dilution of the lysate is used, with a total of 41 plates. After one day incubation, an average of $1.5 \times 10^4$ plaques/plate is observed. The total of $6 \times 10^5$ plaques observed in the 41 plates is a good representation.

All plaques from each plate are transferred to two nitrocellulose filters (Schleicher and Schuell Co.). The filters are dried, washed, baked in vacuum and pre-hybridized as described (see Maniatis et al.). The hybridization is carried out with a nick-translated probe consisting of a DNA fragment isolated from a human urokinase cDNA (Verde A. et al., Proc. Natl. Acad. Sci., 81, 4727-4731, 1984)

E0.1 - Nick Translation

Plasmid pHUK-I (Verde et al., Proc. Natl. Acad. Sci. 81, 4727-4731, 1984) contains a recombinant insert complementary to the mRNA for human urokinase. A fragment of about 1.5 Kb is cut out by treatment with Pst-I restriction endonuclease, isolated by agarose gel electrophoresis, cutting out of the corresponding gel region, and recovering of the DNA fragment by electroelution (c.f. 1.5.7). The 1.5 Kb PstI fragment contains about 75% of the coding region of the human urokinase mRNA (Verde et al., Proc. Natl. Acad. Sci., 81, 4727-4731, 1984). Uniform labeling of this PstI fragment (5.0 microg) is carried out with a nick-translation kit of the Bethesda Research Laboratories in the presence of 200 microCi each of alpha $^{32}$P-dCTP and alpha $^{32}$P-dGTP (c.f. Maniatis et al.). The yield of labelled DNA is 1.8-2.9 x $10^8$ cpm/microg.

E0.2 - Isolation of uPA gene positive phages

The 41 couples of filters are hybridized with the nick-translated Pst-I fragment. Groups of 8 filters are hybridized in 40 ml of hybridization solution in a single plastic envelope with about $10^8$ cpm of labelled DNA. Hybridization is carried out at 42°C for 17 hours in 2 x SSC, 0.1% sodium dodecyl sulfate (SDS)-(Maniatis et al.). The filters are washed four times in 2 x SSC, 0.1% SDS for 15 minutes at room temperature, and once in 1 x SSC, 0.1% SDS for 2 hours at 68°C. After drying, the filters are exposed to X-ray films (Kodak) for 12-24 h and developed. From this screening, the plaques that are found to give a positive hybridization spot in identical positions on both duplicate filters (three) are picked up with a small volume of NZYMC broth (Maniatis et al.) and used to infect E. Coli K 12 L 392 at different dilutions and spread on 10 cm plates of NZYMC-agar. The plates that give between 500 and 5000 plaques are then selected and re-screened These plaques are transferred onto duplicate nitrocellulose filters (Schleicher and Schuell Co.) and the filtered are treated and hybridized with the nick-translated 1.5 Kb Pst-I fragment of human uPA cDNA described above. Practically all plaques give a positive hybridization signal, with an enrichment factor of about $10^5$. Single plaque isolates of the three positive plaques were named lambda-upA-1 lambda-uPA-2 and lambda-uPA-3.

E0.3 - Large scale phage reparation

The plaques are amplified on E. Coli K12 LE 392 using large culture lysates, the phages are isolated by repeated banding in cesium-chloride, purificated by extraction of the aqueous phase with phenol and then with chloroform/isoamyl alcohol (24:1) and DNA ethanol precipitation from the aqueous phase according to the procedure described by Maniatis et al., ch 3.

Example E1

Expression of uPA-coding gene in mammalian cells

E1.1 - Isolation of the uPA ORF

200 microg of lambda-uPA1 obtained essentially as in E0.3 are digested in 80 microl with 30 U of SmaI at 25°C for 1 h; the reaction is stopped with EDTA (final concentration 50mM) and the DNA electrophoresed onto an ethidium bromide containing 0.8% agarose gel using a 10 cm comb at 100V. Then, the gel is analyzed under UV light and the second slowest band, a SmaI fragment containing the open reading frame (ORF) of the human uPA gene, of about 7.3 kb cut out from the gel. The contained DNA is electroeluted and purified as described in 1.5.7.

## E1.2 - Preparation of vector DNA

A RSV-neo derived fragment containing the RSV promoter and the pBR 322 derived ampicillin resistance and origin of replication is prepared by HindIII digestion, filling in, HpaI digestion, CIP treatment and fragment separation on agarose gel.

More particularly, RSV-neo vector is modified as follows (see fig. 2):

## E1.2.1. - HindIII digestion.

20 microg RSV-neo plasmid DNA is digested to completion with 80 U of HindIII for 1 h at 37° C in 200microl. The restriction enzyme is heat inactivated and then the mixture is extracted with an equal volume of phenol: chloroform, the DNA is precipitated adding also 50 microg tRNA (1.5.3), and re-dissolved in 20microl TE (Maniatis et al)

## E1.2.2 - Filling in and HpaI digestion.

The protruding ends of the open vector prepared above are made flush by incubating the DNA with 1U of the Klenow fragment of DNA polymerase I in 25 microl (Maniatis et al, pag. 113) for 1 h at 37° C and purifying it as indicated in 1.5.2 and 1.5.3. Then the linearized, blunt-ended vector DNA is cleaved with 80 U HpaI in 40 microl for 1 h at 37° C and purified in the same manner. These operations cleave the neomycin resistance gene leaving a linear flush-ended 3.5 kb long DNA fragment having the RSV-LTR opened between the promoter region and the polyadenilation site and containing the origin of replication and the ampicillin resistance gene derived from plasmid pBR322 (Fig. 2)

## E1.2.3 - CIP treatment.

The DNA is treated with 1.5 U calf intestine phosphatase (CIP) in 50 microl reaction medium (final volume) in order to remove the 5' phosphate residue from the DNA ends; the enzyme is subsequently inactivated by adding ethylene bis(oxyethylenitrilo) tetracetic acid (EGTA) (final concentration 10 mM) and treatment at 68° C (see 1.5.4.).

## E1.2.4 - Fragment purification.

DNA fragments are separated using a 0,6% agarose preparative gel as indicated in 1.5.7. The portion of the gel containing the 3.5 kb band is cut out and the DNA fragment is electroeluted (1.5.7), the obtained mixture is adjusted to the appropriate salt concentration and purified on a Elutip-D column (Schleicher and Schuell Co.). After elution, ethanol, Na-acetate, and carrier tRNA are added to the DNA solution and the DNA is precipitated and redissolved in 20 microl TE as described in 1.5.3.

## E1.3 - Cloning of the uPA gene under the RSV-LTR promoter

3 U of T4 DNA ligase and 2.5 microl of 10X buffer (Maniatis et al., p. 246) are added to 3 microg of dephosphorilated DNA vector (prepared as described above in E1.2) and 7.5 microg of 7.3 kb uPA fragment (prepared as described in E1.1). The 25 microl reaction mixture is incubated 16 h at 14° C (see 1.5.5) and used to transform 100 microl of HB101 E. Coli competent cells (see 1.5.8). Bacteria are selected overnight on LB agar plates containing 50 microg/ml ampicillin, at 37° C. Isolated colonies are picked up, grown in 5 ml ampicillin containing LB and processed by standard miniprep analysis (1.5.9). The restriction pattern of the purified plasmid DNAs are studied in order to establish the right orientation of the uPA gene in the vector frame. One of these plasmid having the correct orientation is designated RSV-uPA, isolated and used in further experiments (Fig. 2).

### E.1.4.1 - Transfection.

RSP-uPA obtained above is used to transfect mammalian cells. The procedure used is outlined in more details below in example 2 when dealing with the transfection with GASGAs vectors.

### E.1.4.2 - Mass cultivation

Highest producing transformed cells as obtained above are further recloned and mass cultivated in larger fermentors according to the technique reported in the corresponding paragraph of example 2.

### E1.5 - Quantitation of uPA

uPA production by cell lines cultivated as described above is quantified directly on the G418 resistant clones obtained as described above or by spotting serial dilutions of the supernatants of G418 resistant cells on casein/agarose/plasminogen plates as known in the art by evaluating the radial diffusion of the PA, after plasmin activation in case of single-chain one, in a casein/plasminogen containing agar gel substantially as described by Schumaker GFB and Schill W.B., in Anal. Biochem. 48, 9, 1972, and by Bjevum OJ et al, in Quantitative Immunoelectrophresis, Scandinavian J. Immunol. suppl. 2, N.H. Axelsen ed., Aas and Wahl, Oslo, 1975, 82-83. Alternatively, production can be evaluated by IAA (see 1.6.2).
The analysis of activated two-chain uPA versus the corresponding single-chain form is performed by IAA (see 1.6.2).

## Example 2

## Costruction of GASGAs expression vectors

In order to obtain trascription units expressing EO or E2 GASGAs we modified plasmid RSVuPA (see E.1.1.3) by inserting a unique site in intron C (5' from exon IV, that contains most of the EGF-like domain coding region) or in intron D (3' from exon IV) (Fig.3B). The resulting plasmids, RSVuPA$_{P>S}$ and RSVuPA$_{B>x}$ (Fig. 3 c), are used to mutagenize the human uPA ORF by exon shuffling obtaining two GASGAs expression vectors (Fig.3D):
RSVuPA-EO, in which the EGF-like domain containing exon IV has been deleted;
RSVUPA-E2, in which exon IV has been duplicated.

### E2.1 - Costruction of RSVuPA$_{B>x}$

5 microg of RSVuPA DNA are partially digested with BglII in separate reaction vessels with the enzyme ranging from 10U to 0.12U in 12.5 microl of buffer (final volume) for 30 min at 37°C to find the most suitable reaction conditions. The reaction tubes are placed in a dry ice/ethanol bath, EDTA is added to 10mM (final concentration) and finally the enzyme is inactivated by heating for 20 min at 70°C. A sample from each tube is analized on minigel. Good results were found by digesting with 3.33U and 1.11U of the enzyme.
These reaction conditions were chosen and applied to 5 microg of RSV-uPA DNA. The 5' protruding ends thus obtained were separately filled in using 2U of T4 DNA polymerase, in the presence of dNTPs (final concentration 1mM each) in 20 microl (final volume) for 15 min at 37°C. Then, the polymerase is inactivated by heating at 70°C for 20 min. 2 microg of phosphorylated XhoI linker is added and ligated to the plasmid at 8°C for 16 h in 80 microl buffer (final volume) containing 10% PEG 1500 and 1U T4 DNA ligase. After heat-inactivation, 8 microl of XhoI buffer, 80U in 1 microl of XhoI and 11 microl of water are added and the DNA is digested for 3 h at 37°C; the enzyme is then heat-inactivated and the DNA is precipitated, resuspended in 5 microl TE and ligated for 3 h at 15°C with 1U T4 DNA ligase in 50 microl buffer. 2.5 microl of this mixture are used to transform E.Coli HB101 competent cells and ampicillin resistant colonies are screened by miniprep analysis (1.5.9). A plasmid having the BglII site between exons IV and V converted into a unique XhoI site (this modified DNA fragment is illustrated in Fig. 3C, on the right) is isolated and named RSVuPA$_{B>X}$ (hereinbelow referred to also as pB>X)

E2.2 - Construction of RSVuPA$_{P>S}$

Plasmid RSVuPA$_{P>X}$ is obtained by partially digesting RSVUPA with PstI. Restriction conditions leaving approximatively 50% of DNA uncleaved after 1h at 37°C are scaled up to a 50 microg/50 microl digestion (0.0625U/microg) and, after DNA precipitation, the 5' protruding ends are blunted by treatment with 2U of T4 DNA polymerase at 37°C for 10 min in 50 microl (final volume). 5 microg of phosphorylated SalI linker DNA are then added and ligated 16 h at 8°C in 100 microl (final volume) with 2U T4 DNA ligase and 10% PEG 1500. The ligase is heat-inactivated and the DNA is digested (300 microl of buffer final volume) with 80U SalI at 37°C for 3h. Finally the obtained DNA is loaded on a 0.75% agarose gel and run in parallel with undigested and linearized plasmid DNAs (500 ng each). A gel portion containing the bands which run close to the linearized marker is cut out, electroeluted and purified (1.5.7), resuspended in 5 microl TE and self-ligated in 10 microl for 4h at 15°C with 2U of T4 DNA ligase (see Maniatis et al). 2.5 microl are used to transform E.Coli HB101 competent cells and ampicillin resistant colonies are selected by miniprep analysis (1.5.9). A plasmid having a unique SalI site in place of PstI site located between exons III and IV (this modified fragment is illustrated in Fig. 3C, on the left) is isolated and named plasmid RSV uPA $_{P>S}$, hereinbelow referred to also as pP>S.

E2.3 - Preparation of NdeI/XhoI DNA fragments from RSVUPA$_{B>X}$

15 microg of RSVuPA$_{B>X}$ DNA are completely digested with 40U of NdeI for 3h at 37°C in 40 microl (final volume). This volume is brought to 50 microl with 5 microl 10X buffer (Maniatis et al.), 1U of CIP and water. The DNA is dephosphorylated for 60 min at 37°C, extracted once with phenol:chloroform, then precipitated and resuspended in 20 microl TE (see Maniatis et al). The linearized plasmid is then fully digested in 25 microl with 40U of XhoI and run on a preparative 0.75% gel (1.5.7). The two bands which form are separated, electroeluted and purified as described in 1.5.7 and resuspended in 5 microl TE.

E2.4 - Preparation of NdeI/SalI DNA fragments from RSVuPA$_{P>S}$

15 microg of RSVuPA$_{P>S}$ DNA are digested to completion with 40U of SalI at 37°C for 60 min (40 microl, final volume). The reaction volume is brought to 50 microl with 5 microl 10X buffer, 1U CIP and water, and the DNA is dephosphorylated for 60 min at 37°C, purified as described in the previous paragraph, digested to completion in 25 microl with 40U of NdeI at 37°C for 5h and the two resulting DNA fragments are separated and purified as described in 1.5.7.

E2.5 - Construction of RSVuPA-E0

1 microg of the 1.7kb NdeI/SalI fragment from RSVuPA$_{P>S}$ obtained above is ligated with 2.5 microg of the 8.6kb XhoI/NdeI fragment from RSVuPA$_{B>X}$ in 20 microl with 2U ligase at 15°C for 16h. 5 microl of this mixture are used to transform E.Coli HB101 competent cells and, among the ampicillin-resistant colonies, plasmid RSVuPA-E0 is isolated wherein uPA exon IV is deleted by fusing introns C and D and wherein a tyrosine unit which is generated by the fusion of residue base pairs of the codons relative to Ser$_3$ and Asp$_{45}$, is inserted between Pro$_8$ and Lys$_{46}$ (Fig.3D, on the left).

### E2.6 - Construction of RSVuPA-E2

1 microg of the 2.2kb Ndel/Xhol fragment from RSVuPA$_{B>X}$ is ligated to 2 microg of the 9.1kb Sall/Ndel fragment from RSVuPA$_{P>S}$ incubating in 20 microl at 15°C with 2U of ligase. Ampicillin-resistant colonies from E.Coli HB101 competent cells transformed with 5 microl of this mixture are screened by miniprep analysis to isolate plasmid RSVuPA-E2 in which exon IV and part of the flanking introns are duplicated and the two EGF-like domains are connected by an threonine created by the fusion codon (Fig.3D, on the right).

### E2.7 - Alternative method of costructing RSVuPA-E0

An alternative method of costructing E0 GASGAs is to clip out exons III and IV from a plasmid, such as RSVuPA$_{B>X}$, by double digestion with HindIII and Xhol and to substitute them with a HindIII-PstI fragment, encompassing only exon III, wherein the PstI site is conveniently modified to join the Xhol end (see fig. 14). Briefly, a 275bp fragment is obtained from plasmid p71 (fig.5) by:
- total PstI digestion;
- modification of the ends by T4 DNA polymerase and addition of Sall linkers;
- HindIII digestion and CIP tratment followed by Sall digestion;
- gel purification of the fragment.

This fragment was inserted in vector obtained from RSVuPA$_{B>X}$ by:
- Xhol digestion and CIP treatment;
- HindIII digestion;
- gel purification.

### E2.7.1 - Preparation of the exon III containing, 275bp fragment

400 microgram of plasmid p71 is digested 1 h at 37°C with 800U PstI in 350 microliter of buffer (1.5.1), then the DNA fraction is extracted and precipitated (1.5.2 and 1.5.3). A 0.69kb fragment is isolated and purified on 1.2% agarose preparative gel (1:5.7). This fragment is treated with T4 DNA polymerase 45 min at 37°C, the enzyme is then heat-inactivated, the reaction conditions adjiusted to CIP treatement in 100 microl (1.5.4) and the DNA dephosphorylated 30 min at 37°C with 11U CIP, extracted, precipitated and ligated to a Sall 8mer linker, for 16 h at 12°C. After that, the DNA fraction is HindII digested in 200 microl buffer with 400U HindIII at 37°C for 30 min, then further 50U of the enzyme is added and the reaction continued for further 60 min. Then, the enzyme is heat-inactivated, the reaction conditions are adjusted to CIP conditions in 400 ml buffer containing 11U CIP and the DNA is dephosphorylated for 90 min at 37°C. Finally, the DNA is digested with 180U Sall in 200 microl buffer (final volume) for 16 hours at 37°C and the 275bp DNA fragment which is obtained is isolated and purified on a 2% agarose gel.

### E2.7.2 - Preparation of the Xhol-HindIII fragment from RSVuPA$_{B>X}$

50 microgram of the RSVuPA$_{B>X}$ plasmid is digested in 350 microl buffer with 200U Xhol et 37°C for 2 h and 30 min (1.5.1), dephosphorylated in 800 microl buffer with 11U CIP at 37°C for 30 min, then at 50°C for 60 min (1.5.4), and finally extracted and precipitated (1.5.2 and 1.5.3), and fully digested with HindIII at 37°C for 1 hour. The 9.9kb DNA fragment which forms is isolated and purified by a 0.8% preparative agarose gel procedure (1.5.7).

### E2.7.3 - Costruction of RSVuPA-EO-49

The 275bp fragment perpared as in E2.7.1 and the vector DNA obtained as described in E2.7.2 are mixed in a 2:1 molar ratio at different concentrations (approximatively 600ng/ml and 60ng/ml, respectively,) and ligated with 5U T4 DNA ligase at 16°C in 100 microl for 65 h, then the DNA fraction is precipitated and resuspended in 20 microl water. Aliquotes from this solution are used to transform E.Coli DH5 competent cells (1.5.8). Among the ampicillin resistant colonies one was selected by miniprep analysis for having the expected restriction pattern and named RSVuPA-E0-49. (see Fig.14).

### E2.8 - Expression of GASGAs in mammalian cells

GASGAs expression vectors are transfected in mouse LB6 cells by calcium phosphate precipitation techniques and GASGAs-producing clones are analyzed by:
- casein lysis assay, as described by Piperno-Granelli and Reich (J. Exp. Med. 148: 223-234, 1978), in order to select PA producing clones and to quantitize the amount of PA selected in the culture medium;
- fibrin plate method, as described by Astrup T., Mullertz S., Arch. Biochem. Bhiophys. 1952, 40, 346-351;
- IAA, (c.f. 1.6.2) in order to establish the relative amounts of single-chain versus activated, two-chain PAs;
- receptor binding assay (as described by M.P. Stoppelli at. al., Proc. Nath. Acad. Sci. USA, 82, 4939-4943, 1985), to verify the ability of GASGAs to bind to the uPA receptor on U937 cells which is thought to be the same or very similar to the endotelial uPA receptor.

### E2.8.1 - Transfection of GASGAs expression vectors

One day before transfections, the cells (murine LB6, described by Corsaro C.M. and Pearson L.M. in Somatic Cell Genetics, 7, 603, 1981), are plated at a concentration of about $10^4$ microl/$cm^2$ in Dulbecco's modified Eagle medium (DMEM, Flow Laboratories Inc.) supplemented with penicillin (50 U/ml), streptomycin (50 microg/ml), 2 mM glutamine, and 10% heat inactivated foetal calf serum (FCS). The day of transfection, the cells are in logarithmic growth phase and are refeed 3 h before DNA addition. The calcium phosphate-DNA co-precipitation method is essentially followed for transfection (Graham and Van der Eb, Virology 52, 456, 1983). Calcium phosphate DNA precipitate is prepared by adding a DNA solution (in this case about a 1:1 mixture of a GASGAs expression vector, see above, and RSV-Neo, see E1.2) in 2M $COCl_2$ (0.25 ml), (final volume) to a 2 X HBS buffer (137mM NaCl, 0.7mM $Na_2HPO_4$, 21mM HEPES, 4-(2-hydroxyethyl)-1-piperazine ethansulfonic acid, pH 7.1) (0.25 ml). This DNA co-precipitated mixture is then added to the cells. After 3 h at 37°C, the precipitate is removed from the cells which are washed twice with DMEM (without foetal calf serum), 1.5 of a 15% solution of glycerol in HBS is added, the cells are incubated 1-2 min at 37°C and glycerol is removed by washing with DMEM (without foetal calf serum). Then the cells are feeded with complete DMEM (see above when dealing with cell cultivation) and left at about 37°C for about 48 h, trypsinized very gently and plated at a concentration of about $10^5$ cell/plate in DMEM + 10% heat inactivated FCS + 50 U/ml penicillin, 50 microg/ml streptomycin, 2mM glutamin + 0.8 microg/ml G 418 (Geneticin). Then, the cells are incubated for about 2-3 weeks, changing the medium every 3 days.

### E2.8.2 - Selection of GASGAs producing clones

GASGAs clones are selected by the casein or fibrin assays mentioned above (E.2.8). The casein lysis assay is conducted by covering the culture with a casein-plasminogen-agarose overlay prepared for each plate as follow:
- 0.25 ml non-fat, dry milk (8% w/v, heated 30 min in a boiling bath);
- 0.50 ml 2X DME medium;
- 50 microl of 7.5U/ml plasminogen, sterilized on 0.22 micrometer filter;
- 0.2ml agarose from a 5% autoclaved solution.

These components are added to an agarose solution kept in a 48°C bath and layered on the culture cells after the medium has been removed. The cells are incubated at 37°C in a humified, 5% $CO_2$ containing incubator and monitored for the apperance of clear areas in the opaque layer at 2, 4 and 8 hours. Clones that show the highest PA activity are picked up aspiring them with a Pasteur pipette previously filled in with approximatively 0.5% DME, resuspended vigorously and replated in 24 well plates. Then the cells are grown to confluence, trypsinized and 1/5 replated in the same type of culture plates. After 48 hours the supernatants are tested as described below in more details.

The fibrin plate assay is carried out with a similar procedure, but substituting to the reagents listed above a preparation of 2.5 ml/100 mm plate of fibrin-agarose (see Astrup T., the cited reference) in 1 x DME (see above).

E2.8.3 - Quantitation of GASGAs

a) In order to quantitate the PA content, supernatants from GASGAs expressing clones as well as from A431 cells (positive control) are compared with dilutions of a standard concentration of uPA on casein-plasminogen-agarose plates prepared as described in the previous paragraph except that the final agarose concentration was 1.8% and that 5ml were used for each 100mm plate. Holes are made at regular intervals aspiring with a Pasteur pipette. The collected supernatants are serially diluted 1:2 with buffer and 10 microl from each dilution is applied to the plates. The uPA standard solution is first prepared 2X in water and then diluted 1:1 with 2X DME to a final concentration of about 4000 UI/ml. From this solution 1:2 serial dilutions are made in DME and 10 microl of each of them is applied to the plates. GASGAs producing clones are rated in three different classes and GASGAs concentrations inferred by comparison with serial dilutions of a standard uPA preparation.

b) Alternatively, G418 resistant colonies are counted, transferred into 24-well microtiter plates, and allowed to grow up to saturation prior to assaying the supernatants. The clones which demonstrate useful levels of GASGAs production (greater than 0.5 microg/ml) are stored as frozen stocks in liquid nitrogen after supplementing heat inactivated foetal calf serum with 10% DMSO. These cells are also re-cloned to select high producers which are also stored frozen as described above.

The clones which give the highest production of GASGAs are recloned and mass cultivated. The results of experiments using CHO (Chinese Hamster Ovary) or murine LB6 cells are reported below in Table 1:

Table 1

| Cell line | No. of G 418 resistant clones | No. of pro-uPA producing clones | No. of selected clones | GASGAs production (gamma/ml) |
|-----------|------------------------------|--------------------------------|------------------------|------------------------------|
| CHO | 67 | 3 | 1 | 2-10 |
| LB6 | 80 | 55 | 5 | 0.5-1 <br> 1-3 <br> 1-5 <br> 2-10 <br> 5-10 |

E 2.8.4 - Mass cultivation.

A 4 liter cell reactor with automatic control of the dissolved oxygen tension, pH and temperature is used to grow an inoculum of the highest producing clone obtained according to the procedure described in the above examples. The medium for the culture is DMEM with 5% heat-inactivated FCS, 1mm glutamine, 25U/ml penicillin, 25 microg/ml streptomycin and 400 microg/ml G 418 or a serum-free formulation containing DMEM as the base supplemented with albumin, insulin, transferin, ethanolamine, choline, vitamins and trace metals.The cell inoculum is grown in a 150 cm² flasks in DMEM plus 10% heat-inactivated FCS, 2MM glutamine, 50 U/ml penicillin, 50 microg/ml streptomycin, 400 microg/ml G 418, and the cells are cultivated on Cytodex microcarriers (Pharmacia).

The cells attained a maximum population density of $2.5.10^6$/ml and GASGAs are synthesized throughout the duration of the culture and assayed by known methods such as the fibrinolytic or the IAA method (see 1.6.2). A maximum concentration of 20 microg/ml was reached as tested by IAA.

E2.8.5 - Quantitation of activated GASGAs

To quantitate the relative amount of activated, two-chain PAs in cultures of GASGAs-producing clones or upA-producing A431 cells (positive control), supernatants from said cultures are tested in a IAA assay (1.6.1) using mouse monoclonal antibodies which recognize the uPA catalytic domain or Kringle without interfering with the enzymatic activity and without binding the GFD.
The results indicate that generally the amount of two-chain, activated PA ranged between 20% and 50% of the total PA and that it was less then 5% when protease inhibitors were added to the growing cultures.

E2.9 - Receptor binding assay

Supernatants from GASGAs producing clones or from A431 cells are tested for their ability to compete with the binding of radiolabelled $^{125}$I-DFP-UK to the uPA receptor on U937 cells (Stoppelli M.P. et al. Proc. Natl. Acad. Sci, U.S.A, 1985, 82 4939-4943). Colonies which secrete a PA-like active substance are picked up as described in E2.8.2, grown to confluence and the supernatants are tested as described above. High producing clones (see Table 1) and A431 cells are separately plated at $10^5$ cells/ml. PAs concentration is measured by IAA on samples of supernatants using monoclonal antibodies as described in E2.8.5 above. 1:2 Dilutions are prepared in the same medium used for the culture to carry out the receptor binding assay substantially as described by Cubellis et al. (J. Biolog. Chem. 261: 15819-15822, 1986). Briefly, 200 microl of each dilution are used to resuspend $1-2 \times 10^6$ U937 cells (see above) and to each cell suspension an amount of $^{125}$I-DFP-UK having an activity corresponding to about 20000-80000 cpm (final scu-PA concentration of about 50 pM) is added.
The results of a representative experiment are shown in Fig.13 and demonstrate that while supernatants from RSVuPA-EO transfected clones are not able to compete with the iodinated tcuPA for the binding to the uPA receptor.

E2.10 - Purification of GASGA-E0 products

Supernatant from GASGA-E0 producing CHO cells growing in the presence of aprotinin are collected and the GASGA-E0 purified as described in 1.6.5 by immunochromatography, ion exchange chromatography and reverse phase liquid chromatography (1.6.3). (Purity higher than 95% by SDS-PAGE electrophoresis).

E2.11 - GASGAs biochemical and immunological characterization

Purified GASGA-E0 show an apparent molecular weight of about 50kD under reducing and non reducing conditions (1.6.4). After activation by plasmin (1.6.2), the reduced protein is a two-chain protein formed by a 30kD chain that migrates as the B chain of tcuPA and a 20kD chain that migrates faster than the A chain of tcuPA. As expected, when analyzed by immunoblotting the GASGA-E0 protein reacts with mAB 5B4 (recognizing the "kringle" region), with total mouse anti-upA serum but does not react with mABs $DC_1$ or $CD_6$ that recognize the GFD region (see "preparation").

E2.12 - N-terminal aminoacid sequence analysis

The N-terminal aminoacid sequence is performed using an Applied Biosystem model 470 gas-phase protein sequencer obtaining the following result:

```
GASGA-EO     1                 5                          10
    a.a.    Ser Asn Glu Leu His Gln Val Pro Tyr Lys Ser Lys
    uPA      1                 5              8 (24)   46


GASGA-EO               15                      20                 24
    a.a.    Thr --- Tyr Glu Gly Asn Gly?His Phe Tyr Arg Gly
    uPA          50                      55                 60
```

The numbers stand for the GASGA-E0 and uPA sequence; $Tyr_{24}$ in uPA is substituted by $Tyr_9$ of GASGA-E0 as a result of the codon shared between exons III and V in the RSVuPA-E0 recombinant plasmid (see Fig.3) and connects the last aminoacid fully encoded on exon III (uPA $Pro_8$) with the first aminoacid fully encoded on exon V (uPA $Lys_{46}$). Identification of the aminoacid at cycle 19 is not clear and is therefore indicated with a question mark. Cycle 14 did not indicate the presence of any detectable aminoacid suggesting, as expected, the presence of a Cys residue (which is undetectable with the sequentiator used in this analysis unless it is protected in advance by carbomethylation).


## Example E3


### Production of GASGAs by RSV-uPA-BPV vectors


In the RSVuPA plasmid DNA the restriction endonucleases NdeI and ApaI cut only once each (Fig.5), the first in the pBR322 derived region close to the RSV promoter and the second in the SV40 region 3' to the uPA polyadenilation site. These two sites can be modified by XhoI linkering to obtain, after XhoI digestion, a fragment containing the RSV promoter/uPA coding region suitable for cloning in a vector having a XhoI site and/or a SalI site.


E3.1.1 -

70 microg of RSV-uPA DNA are digested to completion with 420 U NdeI in 300 microl for 24 h at 37°C (assay conditions: 150mM Nacl, 10mM Tris.HCl pH 7.8,7mM $MgCl_2$, 6mM 2-mercaptoethanol, 100 microg/ml BSA). After heat-inactivation, the NaCl concentration is adjusted to ApaI conditions (6mM NaCl, 6mM Tris.HCl pH 7.4, 6mM $MgCl_2$ 6mM 2-mercaptoethanol, 100 microg/ml BSA) and the DNA is completely cleaved with 160 U of APaI in 400 microl (final volume) in 4 h at 30°C. The DNA is then purified and precipitated as in 1.5.3, redissolved in 20 microl of TE and the NdeI protruding ends are filled in with 5 U E. Coli DNA Polymerase I incubating the DNA (final reaction volume 25 microl) for 1 h at 37°C followed by heat inactivation of the enzyme (Maniatis et al., pag. 113).


E3.1.2 -

The NdeI/ApaI digested, blunt-ended RSV-uPA plasmid as obtained above is mixed to phosphorilated XhoI linker DNA (molar ratio about 1:15) and incubated 18 h at 8°C, (final volume 50 microl), as indicated in 1.5.5, followed by ligase heat-inactivation. Then the DNA is digested with 120 U of XhoI in 200 microl for 4 h at 37°C and XhoI is heat-inactivated.

E3.1.3 -

The DNA fragments obtained above are separated by electrophoresis on an ethidium bromide containing, 0.75% agarose gel and run 20 h at 35 V (see 1.5.7 for further details), the otained bands are detected on an UV transilluminator, the largest corresponding to the 8.35 kb RSV promoter/uPA coding region fragment and the smallest to the 2.45 kb ampicillin resistance and origin of replication containing-fragment. The two bands are cut out and purified as in 1.5.7. The smallest band is finally treated with 3 U of CIP for 30 min at 37°C and 30 min at 56°C and purified and precipitated as described in 1.5.2 and 1.5.3. The large and the small fragments are mixed in different molar ratios ranging from about 1:1 to about 4:1, ligated 3 h at 14°C and a fraction of each reaction mixture is used to transform E. Coli DHI competent cells as described in 1.5.8. Bacteria are grown overnight on ampicillin containing agar plates, plasmids from isolated colonies are analyzed for the appropriate restriction fragment pattern and one of them selected as plasmid p71, in which the human uPA gene placed under the RSV promoter translational control is excisable from the rest of the plasmid by cleavage with XhoI. (Fig. 5 bottom)

E3.1.4 - BPV-restriction sites modifications

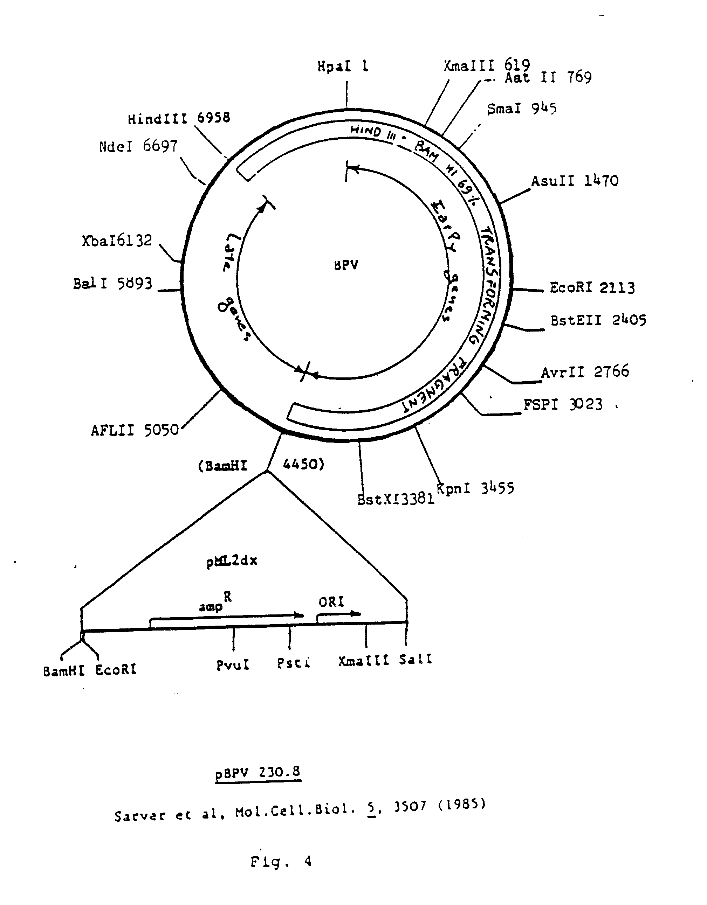

pBPV 230.8 (Fig. 4) is modified at three different sites (BamHI, HInd III or SalI, respectively) by inserting a unique XhoI site that make the insertion of the RSV-promoter/uPAcoding region (RSVuPA) easier. In each experiment, an excess of the appropriate restriction enzyme (BamHI, or HindIII or SalI) is used to digest to completion 10 microg of BPV230.8 DNA for 3 h at 37°C in 100 microl (final volume). The enzyme is heat-inactivated and the DNA is purified as described in 1.5.3, re-dissolved in 10 microl of buffer (Maniatis et al., pag. 113) and rendered flush-ended by treating with 5 U of of the Klenow fragment of E. Coli DNA polimerase I for 2 h at 37°C. After polymerase heat inactivation, the DNA is dephosphorilated in 50 microl (final volume) with 5 U of CIP for 30 min at 37°C and for further 30 min at 56°C. Finally, this enzyme is inactivated by heating in the presence of EGTA and the DNA is purified as described in 1.5.2 and 1.5.3. To the redissolved blunt-ended, linear BPV230.8 DNA, labelled XhoI linker DNA is added (molar ratio 100:1) and ligated in 50 microl (final volume) for 15 h at 8°C in the presence of 2.5 U of T4 DNA ligase (1.5.4). After ligase heat inactivation, a sample containing about 1% of the ligated DNA is digested with 8 U of XhoI, electrophoresed with an equal amount of DNA aliquoted before and after the ligation and analyzed as in 1.5.6. Then, salt concentration is adjusted to 100mM NaCl and the pH is adjusted to about 7.5 (Maniatis et al, pag. 104 and Biochemicals for Molecular Biology; Boehringer Mannheim, Munich 1985), all the DNA is cleaved with XhoI, 1 h at 37°C in 200 microl, loaded on 0.6% agarose gel, electrophoreted and the band corresponding to the linearized plasmid cut out, electroeluted and purified as in 1.5.7. The linear DNA is self-annealed by ligation and used to transform competent E. Coli DH5 cells (BRL) (see 1.5.8). Plasmids from colonies isolated after overnight incubation on ampicillin containing agar plates are screened by miniprep analysis (1.5.9) for the presence of the new XhoI site. The following pBPV230.8 derived plasmid were obtained (Fig. 6, in the middle):

-pBB, wherein a XhoI site is created at the BamHI position and is flanked by two BamHI sites;
-pBS, wherein a XhoI site is created at the SalI position and is flanked by two SalI sites;
-pBH, wherein a XhoI site is created at the HindIII position.

E3.1.5 -

In different experiments, 50 microg each of pBB, pBH and pBS DNAs are digested to completion with an excess of XhoI (SalI in the case of pBS) in 50 microl, at 37°C for 2 h, the enzyme is heat-inactivated, the reaction conditions are modified with concentrated buffer (1.5.1) as required to double the reaction volume and the linearized plasmid DNA is dephosphorilated with 1U CIP as described in 1.5.4. After enzyme heat-inactivation, the DNA is loaded on a ethidium bromide containing, 0.6% agarose gel and electrophoreted for 16 h at 30V. A major DNA band corresponding to the monomeric linear plasmid is electroeluted, purified and re-dissolved as described in 1.5.7. Similarly, 50 microg of p71 plasmid DNA is digested to completion with 80U of XhoI, the enzyme is heat-inactivated, and the mixture is loaded on an ethidium bromide containing 0.6% agarose gel and electrophoreted for 16 h at 30 V. The gel portion with the 8.35kb band, which contains the RSV promoter/uPA coding region construct, is electroeluted, purified and redissolved as described in 1.5.7. BPV vector DNA (Fig. 6, in the middle) and insert DNA (Fig. 5, bottom) are mixed in ratios varying from 2:1 to 1:4 and ligated with 2U/sample of T4 DNA ligase for 24 h at 15°C at a DNA concentration of approximatively 0,2 microg/microl. The reaction medium is adjusted with the buffer and the ligated DNA is digested 1 h at 37°C with 20 U of XbaI in 15 microl (final volume). (Assay conditions: 50mM NaCl, 6mM Tris-HCl pH 7.9, 6mM MgCl₂, 100 microg/ml BSA). This enzyme does not cleave the RSV/UPA construct and cut the BPV230.8 derived vectors only once. After XbaI heat-inactivation the obtained DNA mixture is diluted 1 to 5 with water, 10x ligation buffer is added, and incubated 2 h at 14°C with 2.5 U of T4 DNA ligase. A 5 microl sample from each reaction experiment is used to transform E. Coli DH5 competent cells (1.5.8). These bacteria are grown overnight on ampicillin-containing agar plates at 37 C and samples from the resulting colonies are analyzed by miniprep analysis (see 1.5.9). Plasmids from isolated colonies with the proper restriction pattern are selected (1.5.9, Fig. 6, bottom).

### E3.2 -Modification of the uPA trascription unit in BPV-derived expression-vectors

In the uPA ORF containing 7.3 kb SmaI fragment the restriction enzymes BssHI and SacI cut only once each generating respectively a 5′ protruding end at nt 413 and a 3′ protruding end at nt 4344. This fragment comprises the GFD coding region of uPA, while the corresponding fragments in the RSVuPA derived GASGAs plasmids (see Fig. 3) contains the $GFD_0$ and $GFD_2$ regions. The BssHI/SacI fragment in all the BPV-vectors is thus substituted with the BssHI/SacI fragment from RSVuPA-E0 or RSVuPA-E2 to obtain:
- the vectors of the E0-series, having an exon IV-deleted ORF;
- the vectors of the E2-series, having an exon IV-duplicated ORF.
Briefly, the two series are prepared as follow:
a) the DNA from RSVuPA-E0 or RSVuPA-E2 is completely digested with BssHI, treated with CIP, completely digested with SacI and the smallest DNA fragment is purified on gel;
b) the DNA from the BPV-derived uPA expressing vectors is analogously completely digested with SacI, treated with CIP, completely digested with BssHI and the largest fragment is purified on gel;
c) the exon IV-deleted or the exon IV-duplicated BSSHI/SaCl fragments are inserted in each of the above obtained episomal vector DNAs.

### E3.2.1 - Preparation of "E0" or "E2" BssHI/SacI cassettes

50 microg of plasmid DNA from RSVuPA-E0 or RSVuPA-E2 (fig.3) are digested to completion with 64U of BssHI in 75 microl (final volume) for 2 hours at 37°C. The enzyme is then heat-inactivated and the DNA is dephosphorylated with 2.5U CIP in 150 microl (final volume). DNA is then phenol/chloroform purified (1.5.2), precipitated (1.5.3), and resuspended in 100 microl buffer to digest it with 80U of SacI at 37°C for 3 hours. A fragment containing the "E0" cassette or a fragment containing the "E2" cassette are obtained, respectively, and purified on agarose gel (1.5.8).

### E3.2.2 - Preparation of BssHI/SacI BPV-derived vector frames

20 microg of DNA from each pBPV230.8 derived, uPA expressing vectors (see fig. 6, bottom) are completely digested with 40U of SacI for 3 hours at 37°C in 50 microl (final volume), the enzyme is then heat-inactivated and the DNA is dephosphorylated in 100 microl (final volume) with 2U CIP (1.5.4). After extraction and precipitation (1.5.2, 1.5.3), the DNA is redissolved in 100 microl of buffer and digested with 40U of BssHI at 37°C for 2 hours. The biggest fragment which is deleted in the BssHI/SacI EGF-containing region, is isolated on agarose gel (1.5.8).

### E3.2.3 - Insertion of "E0" or "E2" cassettes in the BPV/RSV/uPA frame

This procedure is used to generate the $GFD_0$ ("zero") or the $GFD_2$ ("two") series in BPv-derived, uPA expressing vectors:
a) 1 microg of BssHI dephosphorylated/SacI DNA fragment, containing either the $GFD_0$ or the $GFD_2$ cassette prepared as described above (E3.2.1) is ligated to 4 microg of SacI dephosphorylated/BssHI vector DNA containing the BPV/RSV/uPA frame (E.3.2.2) for 16 hours at 15°C with 2U DNA ligase in 20 microl (final volume);

b) 5 microl of this mixture are used to transform DH5 E.Coli competent cells;

c) ampicillin resistant colonies are screened by miniprep analysis (1.5.9) and the plasmids showing the expected restriction pattern are isolated.

By repeating this procedure starting from each pBPV230.8-derived scu-PA-producing series of plasmids (fig. 7A), two series of BPV-derived GASGA expression vectors are obtained:
- series "zero" wherein at least a portion of the EGF-like domain has been deleted (suffix E0); fig. 7B)
- series "two" wherein most of the EGF-like domain has been duplicated (suffix E2; fig. 7C).

### E3.3 - Expression and analysis of GASGA products

The BPV derived GASGA expression vectors obtained above are cotransfected with RSV-Neo in LB6 mouse cells or CHO cells (see E.1.4) and the G418 resistant clones are selected and analyzed as described in E2.8. PA enzymatic properties, immunological characteristics and uPA receptor binding properties of the obtained proteins reflected that obtained with RSVuPA-E0 and RSVuPA-E2 (see above).

### Example E4

Construction of modified derivatives of the human uPA promoter region.

### E4.1 - Construction of the puPA2 plasmid

50 microg of lambda-uPA1 DNA prepared as described in E0.3 are digested in 100 microl (final volume) with 30 U of SmaI in the appropriate buffer for 60 min at 30°C. 500 ng of the obtained DNA are analyzed on an Ethidium Bromide containing 1% agarose gel while the remaining is stored at -20°C. The enzyme is then heat-inactivated at 68°C (10 min) and the resulting DNA preparation is loaded on an ethidium bromide containing, 0.8% preparative agarose gel. The electrophoretic separation is runned 20 h at 50 V in TBE buffer (Maniatis et al, ch. 5) and the DNA bands are visualized on a medium-lenght UV transilluminator. A band of approximatively 2.38 kb that contains the pro-uPA promoter is cut out and the DNA electroeluted and purified as described in 1.5.7. 50 microg of pEMBL8CAT DNA (Fig. 8) is similarly digested for 1 hour at 30°C with 30 U of SmaI in 25 microl (final volume). 64 Microl of water, 10 microl of CIP 10X buffer and 1 U of CIP in 1 microl (final volume) are added and the reaction mixture is further incubated for 60 min at 37°C. CIP is inactivated adding EGTA and heating as described in 1.5.4 and the DNA is purified on an ethidium bromide containing, 0.8% agarose gel under the same conditions described in 1.5.7. 1.5 microg of the 2.38 kb lambda-uPA1 derived fragment and 500 ng of linearized, dephosphorylated pEMBL8CAT DNA as prepared above are mixed togheter and ligated in 20 microl of the appropriate buffer containing 10% PEG and 2 U of T4 DNA ligase for 20 hours at 14°C. 5 microl of this reaction mixture are then used to transform competent HB101 E.Coli cells as described in 1.5.8. Ampicillin resistant colonies are analyzed, and among those containing the insert in the correct orientation one is selected and designated puPA2. (Fig. 8 on the right).

### E4.2 - Construction of the puPA2/41 plasmid

Optimal conditions to obtain a SmaI partially digested, linearized form of puPA2 DNA prepared above are investigated by digesting 250 ng of this plasmid respectively with 1.0, 2.0, 3.0 and 4.0 U of SmaI in 10 microl (final volume) of the appropriate buffer. 50% (v/v) each reaction mixture is transferred to a different tube and frozen in a dry ice/ethanol bath after 6 min at 30°C of reaction, while the remaining portions are frozen after incubating 9 min longer. Then, each frozen sample is brought to 10 mM with EDTA, the enzyme is heat-inactivated and the DNA is analyzed on an ethidium bromide containig, 1% agarose minigel. Good results are achieved by incubating with 2 U for 15 min and with 3 U for 6 min. Then a preparative digestion is set up as follow: 5 microg of puPA2 DNA is digested with 40 U of SmaI in 50 microl of the appropriate buffer, the reaction is stopped by adding 1 microl of 500 mM EDTA and freezing. After heat-inactivation of the added enzyme this puPA2 DNA preparation is mixed with 2.25 microg of XhoI linker, phosphorylated as described in 1.5.6 and ligated in 86 microl of a reaction mixture containing 10% PEG 1500 and 2.5 U of T4 DNA ligase, incubating first 6 h at 7° C then increasing slowly the temperature to 17° C and incubating for further 10 hours. The ligating enzyme is heat-inactivated, 10% of the ligated DNA is digested with 55U of XhoI for 2 h at 37° C in 20 microl (final volume), this enzyme is then heat-inactivated and the DNA precipitated as indicated in 1.5.3. The precipitated DNA is re-dissolved in 4 microl TE (Maniatis et al), 1 microl of 1U T4 DNA ligase containing 5X ligation buffer (Maniatis et al,) is added, the mixture is left to self-ligate at 15° C for 2h30 min and then used to transform HB101 competent E.Coli cells (1.5.8). Ampicillin resistant colonies are screened by miniprep analysis. One of these, having the SmaI site, which flanks the 5' end of the uPA promoter containing the 2.38kb insert, converted into an XhoI site (Fig. 10) is named puPA2/41.

### E4.3 - CAT analysis

puPA2, its derivative puPA2/41 and RSV-CAT are tested for their ability to drive the trascription of the CAT gene. Plasmid DNAs are transfected into human A1251 cell-line (1.1) which was derived from a kidney carcinoma (Stoppelli M.P., Verde P., Grimaldi G., Locatelli E.K., Blasi F., J. Cell Biol. 1986, 102, 1231-1241) by calcium phosphate precipitation procedure and the levels of the CAT protein possibly produced in the transfected cells are measured after 64 ± 4 hours.

### E.4.3.1 - Purification of plasmid DNA.

Plasmid DNAs are purified using the alkaline lysis method as described by Birnboim H.C. and Doly J. 1979, Nucleic Acids Res. 7, 1513 (reported also in Maniatis et al. p. 90), from a 400ml LB broth overnight culture of each bacterial clone. The plasmid DNA thus obtained is purified twice on ethidium bromide CsCl gradients, the band containing it is collected by punturing, and extensively extracted with ethidium bromide containing isoamylic alcohol (see Maniatis et al. p.93-94). The DNA containing CsCl aqueous solution is then diluted 1:4 with water and the DNA is precipitated by adding 0.1 vol of 3M NaAcetate pH5.2 and 3 vol of ethanol. After leaving the mixture 30 min at -20° C, the DNA fraction is spinned down at 10000XG for 15 min at 4° C, washed once with 70% ethanol and once with 100% ethanol, dried, dissolved in 2ml of TE, reprecipitated and washed in the same manner and finally re-dissolved in 1ml TE (Maniatis et al). O.D.$_{260}$ is measured and the concentration is adjusted to about 500ng/microl, checked on minigel and measured by spectrophotometry again. Finally the plasmid DNAs are diluted 1:1 with absolute ethanol and stored at -20° C.

### E.4.3.2 - Precipitation by calcium phosphate procedure.

For each 60mm Petri dish to be transfected, 40 microl of DNA Ethanol/TE solution, equivalent to 10 microg DNA, are dried in a Savant vacuum centrifuge and re-dissolved in 220 microl of water. 30 microl of 2M CaCl$_2$ (from Mallinkrodt or Merck) are added and quickly mixed. 250 microl of 2X HBS (10g/l HEPES, 4-(2-hydroxyethyl)-1-piperazine ethanesulfonic acid, 16g/1 NaCl, pH 7.1 ±0.05, sterile) and 5 microl of 100X PO$_4$ (70mM Na$_2$HPO$_4$ and 70mM NaH$_2$PO$_4$, sterile) are mixed and the DNA/CaCl$_2$ solution is slowly added dropwise while extensively bubbling in with a Pasteur pipette. This mixture is then left at room temperature for 30 min (Graham and Van der Eb, Virology, 52, 456; 1984).

### E.4.3.3 - Transfection of A1251 cells.

A1251 cells are grown close to confluence in complete medium [DME medium (Gibco) supplemented with 10% fetal calf serum (Hyclone) and 1% antibiotic solution (Gibco)] in 5% CO$_2$ humified incubator at 37° C. 24 hours before transfection, the cells are splitted in 60mm Petri dishes at 10$^5$ cell/dish and re-feed with fresh medium 4 to 6 hours before transfection. At t$_0$ the precipitated DNA is added and the mixture is left for 16 to 18 hours in the incubator. After overnight incubation, the cells are washed once with DME (7.2) then re-supplemented with the complete medium, left in the incubator to complete the incubation and harvested. In the experiments run in triplicate, all the steps following the dissolution of the dried DNA are performed separately for each sample. The harvested cells are used in the subsequent steps.

### E4.3.4 - Preparation of cell lysates.

64 ±4 hours after the addition of the precipitated DNA, the cells (prepared as in E 3.3.3.) are washed 3 times with a phosphate-buffered saline (PBS) free from $Ca^{++}$ (80 g NaCl, 2 g KCl, 11.5 g $Na_2HPO_4.2H_2O$, 2 g $KH_2PO_4$ per 1 liter of distilled water, brought to pH 7.2, then 1ml of STE (100mM NaCl, 10mM TRIS, 1mM EDTA) is added and the mixture is left at room temperature for 5 min. Afterwards the cells are scraped off with a rubber policeman and put in an Eppendorf tube, spinned down in microcentrifuge 3 min at 4°C and resuspended in 100 microl of TRIS 250mM pH 7.8. The cell suspension is left 15 min in a dry ice/ethanol bath and then transferred to a 37°C bath; this freeze/thaw cycle is repeated twice, the cell nuclei are spinned down in microcentrifuge at 4°C for 5 min and the supernatant is transferred to a fresh tube. In order to overcome minor differences in the protein content of each sample due to a possible slight difference in the cell number from plate to plate, protein concentration is determined by evaluating 10 microl from each sample according to the method of Lowry (see E.1.6). The remaining part of the lysates are stored at -20°C until used in the CAT assay.

### E4.3.5 - CAT assay.

A volume containing 100 microg of protein from each lysate is brought to 133.5 microl with water and 44 microl of a premixed solution containing 22.5 microl of 2M Tris pH 7.8, 20 microl of a freshly prepared solution of 3 mg/ml acetylCoA (Sigma) and 1.5 microl radioactive $^{14}C$-chloramphenicol (50-60 mCi/mmol, from Amersham) are added thereto. The mixture is incubated at 37°C for 1 h, then the reaction is stopped by adding 1ml of ethyl acetate and vortexing. The two phases which form are separated in an Eppendorf microcentrifuge (5 min) and the organic phase is transferred to a fresh Eppendorf tube, essicated in a Savant vacuum centrifuge, redissolved in 20 microl of ethyl acetate and assayed by TLC on 0.25-1 mm silica gel plates (Kodak). The chromatography is run for 2-3 h with chloroform:methanol (HPLC grade), 95:5 as the mobile phase, and the radioactive spots corresponding to the labelled antibiotic are visualized by autoradiography. The areas of the chromatogram corresponding to the radioactive spots are cut out, placed in a vial with 5ml of a suitable scintillation solution such as Econofluor, (New England Nuclear) and the radioactivity is measured in a beta-counter. The results are shown in Tab. 2 (see E4.4.3).

### E4.4.1 - Construction of plasmid puPA2/17.

300ng of puPA2/41 DNA (see E3.2) is completely digested with 4.2 U of OxaNI for 4 h at 37°C in the appropriate buffer (100 mM NaCl, 10mM Tris.HCl pH 7.5, 10 mM $MgCl_2$, 10 mM 2-mercaptoethanol, 100 microg/ml BSA). DNA restriction is checked on an ethidium bromide containing minigel (1.5.1) and the enzyme is inactivated by heating 10 min at 68°C. 2 microl of water, 1.5 microl of 10X buffer and 1.5 microl of T4 DNA ligase are then added and the mixture incubated at 15°C for 18 h. 2.5 Microl of this mixture are used to transform E.Coli HB101 competent cells (1.5.8). Ampicillin resistant colonies are analyzed by miniprep analysis (1.5.9). Those having the correct restriction pattern are selected and one of them was named puPA2/17. (Fig. 10).

### E4.4.2 - Construction of plasmid puPA2/254.

4 microg of puPA2/41 DNA (see Fig.10) is digested to completion with 5U of EcoRV in 10 microl of the appropriate buffer (Maniatis et al) at 37°C for 2, h then a mixture of 7 microl of water, 2 microl of 10X buffer and 1U CIP in 1 microl of buffer (Maniatis et al) is added. The mixture is incubated 1 h at 37°C, EGTA is added to a final concentration of 10mM and the mixture is incubated at 68°C for 45 min. The DNA is precipitated, washed and dried as described in 1.5.3 and finally redissolved in 7.5 microl TE (Maniatis et al). The DNA is completely digested with 4.2 U of OxaNI for 4 h at 37°C in 10 microl (final volume) and the 5′ ends are filled in with 1 U of the Klenow fragment of DNA PolI (20 microl, final volume) at 37° C for 1 h. The DNA is then precipitated (1.5.3) and resuspended in 7.5 microl TE (Maniatis et al.). 0.5 microl of 100mM ATP, 1 microl 10X buffer (Maniatis et al) and 2.5U of T4 DNA ligase (1 microl) are added and the mixture is incubated for 18 h at 15° C. Then 2.5 microl of this mixture is used to transform E.Coli HB101 competent cells (1.5.8), the bacteria are plated on ampicillin containing LB plates (Maniatis et al). The colonies having the correct restriction pattern are selected and one of them was named puPA2/254 (Fig. 10).

### E4.4.3 - CAT analysis.

puPA2/41, its deletion derivatives puPA2/17 and puPA2/254 as well as RSV-CAT are tested for their ability to drive the trascription of the CAT gene. Plasmid DNAs are transfected in A1251 and Hela cell lines (1.1) by means of the calcium phosphate precipitation technique and the CAT intracellular level is measured after 64 ±4 hours. Purification of plasmid DNA, precipitation by calcium phosphate, cell transfection, preparation of cell lysates and CAT assay are performed substantially as described in E3.3. The results are shown in Table 2.

TABLE 2

|  | A1251 | Hela |
|---|---|---|
| RSV-CAT | 100 | 100 |
| puPA2/41 | 128 | 32.3 |
| puPA2/17 | 303.8 | 74.3 |
| puPA2/254 | 378.5 | 174.3 |

### E4.5 - Construction of uPA derived expression vector.

The bacterial CAT gene may be excised from plasmids puPA2/41, puPA2/17 and puPA2/254 in order to use them as expression vectors. Preferentially, modifications will conserve the uPA promoter derived sequence, the uPA TATA box, the mRNA starting point, and the pEMBL8-derived polylinker. DNA fragments comprising these regions may be excised from the vector and inserted upstream to different genes as "expression cassettes". From the above described plasmids three uPA-derived expression vectors were obtained by exciding the bacterial CAT gene: pPP41 from puPA/41, pPP17 from puPA2/17; pPP254 from puPA2/254.

### E4.5.1 - Construction of pPP41

25 microg of puPA2/41 DNA are partially digested with 4U of XbaI at 37° C for 45 min and the reaction stopped adding EDTA (to 50mM) and heat inactivating. After precipitation (1.5.3), the DNA is filled in with 2.5U of the Klenow fragment of DNA PolI (final volume 20 microl) at 37° C for 1 h, ligated to a 50 molar excess of SalI linker (1.5.5 and 1.5.6) for 16 h at 8° C with 2U ligase and, after heat-inactivation of the enzyme, completely digested at 37° C for 2 h with an excess of SalI. DNA fragments are run on a 0.75 agarose gel (1.5.7) and the gel portion containing a DNA fragment of 5-6kb range is cut out. This DNA is then extracted and purified as described in 1.5.7, resuspended in 10 microl TE and 2 microl of this solution are self-ligated at 16° C for 4 h with 2U ligase (final volume 10 microl). 2.5 microl of this DNA preparation are then used to transform DH5 E. Coli competent cells. (1.5.8 - 1.5.9). Among the ampicillin-resistant colonies plasmid pPP41, i.e. a plasmid having the region comprised between the SalI site in the polylinker and the XbaI site downstream the CAT gene deleted, is selected by miniprep analysis. (Fig. 11B).

### E4.5.2 - Construction of pPP/17 and pPP/254

Substantially following the above outlined procedure, two expression vectors are obtained by completely digesting 10 microg of puPA2/17 DNA or 10 microg of puPA2/254 DNA, respectively, with 24U XbaI at 37°C for 2 h. The 5´protruding ends of the linearized plasmid are filled in with the Klenow fragment of DNA PolI (2.5U) at 37°C for 1 h and then ligated to a 50 molar excess of SalI linker (1.5.5 and 1.5.6) for 16 h at 8°C with 2U ligase and, after heat-inactivation of the enzyme, the DNA is completely digested at 37°C for 2 h with an excess of SalI. Then, this enzyme is heat-inactivated and the DNA is precipitated (1.5.3) and re-suspended in 10 microl TE. 2 microl of this DNA solution are ligated at 15°C for 16 h with 2U DNA ligase (final volume 10 microl) and 2.5 microl are used to transform DH5 E.Coli competent cells. Among the ampicillin resistant colonies, plasmid pPP17 and pPP254 are selected which carry uPA-derived deletion promoters and have the same general characteristics described for pPP41 in E4.5.1 (Fig. 11B).

## Example E5

### E5 - Production of GASGAs by uPA derived, GFD modified minigenes

### E5.1. - Purification of the uPA containing SmaI fragment

A 7.3kb SmaI fragment containing the uPA coding region was obtained from phase lambda-uPA1 as described in E.1.1 (Fig. 1).

### E5.2 - Preparation of vector DNAs

20 microg each of plasmid DNAs from pPP41XS, pPP17XS and pPP254XS are prepared separately digesting them with 50U of SmaI at 25°C for 3 h (50 microl, final volume), then the linearized plasmids are dephosphorylated with 1U of CIP at 37°C for 1h (150 microl, final volume), and gel purified on a 0.8% agarose gel as described in 1.5.3. The separated DNA preparations are resuspended in 20 microl TE and stored at 4°C.

### E5.3 - Constructions of uPA derived minigenes.

Each linearized dephosphorilated plasmid DNA obtained above (E5.1) (3 microg) is mixed with 1 microg of uPA-ORF (i.e. SmaI fragment of about 7.3. kb containing the uPA coding region obtained as described in E1.1) in 10% PEG 1500 with 2U ligase (final volume 10 microl) for 16 h at 15°C.

2.5 microl from each reaction mixture is used to transform competent E. Coli DH5 cells (c.f. 1.5.8). Among the ampicillin-resistant colonies plasmid pPP41XS, pPP17XS, and pPP254XS, which derive from pPP41, pPP17 and pPP254, respectively, are selected by miniprep analysis (c.f. 1.5.9) for having the following characteristics (see fig. 11C):

- the uPA coding region is inserted in the SmaI site of the uPA promoter containing vector
- the uPA mRNA starting site is the same as the human uPA gene
- it contains a pEMBL8 derived sequence
- the uPA-derived minigenes can be excised from that vectors and inserted in different vectors by digestion with XhoI and SalI
- the excised minigenes can be inserted in vectors having either a SalI or a XhoI site
- the excised minigenes can be inserted in vectors able to maintain them in an episomal form when inserted in a suitable host
- pPP41XS has the uPA coding region under the control of a 2.35 Kb fragment from the uPA promoter;
- pPP17XS has the uPA coding region under the control of the same promoter fragment contained in pPP41XS but in which a deletion has been created between the two OxaNI sites;
- pPP254XS has the uPA coding region under the control of same promoter fragment contained in pPP41XS but in which a deletion has been created between the 5′OxaNI site and the EcoRV site.

### E5.4 - Costruction of GASGAs expressing minigenes

A substitution of a BssHI/SacI DNA fragment, encompassing the uPA ORF from exon II to intron J (c.f. Fig.3A) in the pPP derived expression vectors is made essentially as described for the generation of GASGAs expressing, BPV derived plasmids in example E3.

### E5.4.1 - Purification of vector DNA

20 microg of DNA from plasmid pPP41XS, pPP17XS or pPP254XS is digested with SacI, treated with CIP and restricted with BssHI as described in E3.4.3. The vector frames containing the pEMBL8 region, the uPA promoter, or the derivatives thereof, and the 5′ and 3′ ends of the uPA ORF (c.f. Fig.12A) are purified on agarose gel (1.5.8) and the DNA fragment resuspended in 10 microl TE.

### E5.4.2 - Purification of insert DNA

50 microg of DNA from plasmid RSVuPA-E0 or plasmid RSVuPA-E2 are digested with BssHI, dephosphorylated with CIP and restricted with SacI as described in E3.4.1. The two fragments, containing the "E0" or the "E2" coding region, are purified on agarose gel (see 1.5.8) and resuspended in 20 microl TE.

### E5.4.3 - Construction of GASGAs expressing minigenes

For each derived plasmid the same procedure is used:
a) mixing a vector DNA (prepared as described in E5.4.1) and an insert DNA (prepared as described in E5.4.2) in a 1:2 molar ratio (0.3 microg DNA in 10 microl, final volume) and ligated at 15°C for 16 hours with 2U T4 DNA ligase;
b) transforming DH5 E.Coli competent cells (1.5.8) with 2.5 microl from each of the mixtures obtained above;
c) ampicillin-resistant colonies were analyzed (1.5.9) for having the BssHI/SacI insert, obtaining two series of three plasmids (Fig.12B and 12C).

### E5.5 - Expression and analysis of GASGA products

uPA minigenes derived GASGA expression vectors obtained as described above are cotransfected with RSV-Neo in LB6 mouse cells and CHO cells and the G418 resistent clones are selected and analyzed as described in E2.8.
PA enzymatic properties, immunological characteristics and uPA receptor binding properties parallel that obtained with RSVuPA-EO and RSVuPA-E2.

### Example E6

### Production of "interrupted" GASGAs

33

E6.1 - Restriction site modification: partial digestion

The plasmid RSVuPA contains two ScaI sites, the first in the pBR322 derived ampicillin resistance gene and the second in the GFD, which is contained in exon IV of the uPA gene (see fig.2).

To find out the optimal conditions to linearize RSVuPA, 100 ng of plasmid are digested with ScaI in 10 microl of buffer at $37^\circ$ C for 1 hour (see 1.5.1). The amount of enzyme added is from 10U to 0.3U. Optimal results are obtained with 5 and 2.5U/100ng. Reactions are scaled up to 1 microgram for both concentrations, then, after digestion, EDTA is added up to a final concentration of 10mM, the reaction mixtures are pooled and the DNA extracted and precipitated (see 1.5.2 and 1.5.3). This plasmid DNA is ligated to a 8mer phosphorilated KpnI/Asp718 linker (see 1.5.6) (Boehringer; sequence CGGTACCG) with a molar ratio plasmid/linker 1:100 in 20 microl of buffer (final volume) for 48 hours with 5U T4 DNA ligase (see 1.5.5). The enzyme is then heat-inactivated and the DNA is digested in 400 microl buffer with 360U Asp718 (see 1.5.1), precipitated (see 1.5.3) and loaded on a 0.8% preparative electrophoresis gel run 16 hours at 40V (see 1.5.7). The DNA band corresponding to the plasmid linear size (10.8 Kb) is electroeluted and purified as in 1.5.7, quantitated on minigel and 50ng of it is self-ligated with 2.5U ligase in 24 microl of buffer, precipitated and used to transform 100 microl of competent E.Coli DH5 cells (see 1.5.8). Among the ampicillin resistant clones one, having the expected restriction pattern, was selected and named RSV-G-Asp (see fig. 15).

In order to obtain interrupted GASGAs, RSV-G-Asp can be modified as described below for plasmid RSV-uPA-Tet-Asp (E6.2) by complete filling of the Asp718 site (as described in E6.3.1) or by its partial filling followed by blunting (as described in E6.3.2) thus obtaining two vectors expressing GASGAs products which are equivalent to those obtainable by expressing RSV-SVRTD (E6.3.1) or RSV-SAD (E6.3.2).

E6.2 - Restriction site modification: total digestion

In order to obtain a plasmid carrying a unique ScaI site the ampicillin resistence for the tetracicline resistance (see Fig. 16) is substituted by:
- deleting most of the ampicillin resistance gene in pBR322, obtaining the $Amp^S$, $Tet^R$ plasmid pBR13Tet;
- substituting the HpaI-NdeI fragment in RSVuPA with the NdeI-PvuII fragment from the deleted pBR322.

In the obtained plasmid the ScaI site is present only in the uPA coding region in exon IV and is mutated by insertion of a number of linkers.

E6.2.1 - Costruction of a deleted pBR322 (pBR13Tet)

100ng of pBR322 DNA is digested with 10U of PstI (1.5.1) 1hour at $37^\circ$ C in 50 microl buffer (final volume), the enzyme is then heat-inactivated and the reaction conditions are adjusted to a digestion with 50U of HindIII for 1 hour at $37^\circ$ C in 400 microl buffer, final volume (1.5.1). The DNA fraction is extracted and precipitated (1.5.2 and 1.5.3), redissolved and blunt-ended using 4U T4 DNA Polymerase (Maniatis et al) at $37^\circ$ C for 30 min. After extraction and precipitation the DNA is self-ligated with 2.5U T4 DNA ligase (100 microl buffer, final volume) precipitated and used to transform E.Coli DH5 competent cells. Among the tetracycline positive colonies one is selected by miniprep analysis for having the correct restriction pattern and named pBR13Tet (fig. 16 b).

E6.2.2 - Substitution of $Amp^R$ for $Tet^R$

a) 2 microgram of pBR13Tet is digested in 50 microl buffer (final volume) with 12U of NdeI at $37^\circ$ C for 1 hour, then the reaction conditions are trimmed to 2U CIP treatment (1.5.4) in 200 microl at $37^\circ$ C for 30 min, EGTA is added to a final concentration of 10mM, the DNA fraction is extracted, precipitated, and digested 1 hour at $37^\circ$ C with 20U PvuII in 100 microl buffer (final volume), then extracted and precipitated again and finally redissolved in 10 microl TE.

b) 20 microgram of RSVuPA is digested in 150 microl with 100U of HpaI at $37^\circ$ C for 1 hour, then the reaction conditions are trimmed to 22U CIP treatment (1.5.4) in 300 microl buffer at $37^\circ$ C for 30 min, EGTA is added to a final concentration of 10mM, the DNA fraction is extracted, precipitated digested 1 hour at $37^\circ$ C with 60U NdeI in 200 microl, extracted and precipitated again and finally redissolved in 20 microl TE.

c) Three different ligation reactions are performed (1.5.5) adding to 2.5 microl of restricted pBR13Tet (see E4.4 a) different amounts of digested RSVuPA (see E4.4 b) to obtain molar ratios of 1:1, 1:2 and 1:4. Ligation is performed with 5U T4 DNA ligase in 50 microl buffer, overnight. The enzyme is then heat-inactivated, the DNA fraction is precipated, redissolved in 10 microl water and 5 microl of this mixture is used to transform E.Coli DH5 competent cells. Among the tetracycline positive colonies one was selected by miniprep analysis for having the correct restriction pattern and named RSVuPA-Tet (Fig.16 d).

E6.2.3 - Modification of the Scal site

1 microgram of RSVuPA-Tet is completely digested with 100U Scal in 200 microl at 37° C for 3 hours (1.5.1). The DNA is extracted, precipitated and redissolved (1.5.2 and 1.5.3) in 20 microl TE. In separated reaction vessels, 2 microl of linearized RSVuPA-Tet thus obtained is ligated to a 50 molar excess of the following linkers that do not contain any of the sites which are present in the plasmid before treatment:
- Asp718 8mer (sequence CGGTACCG, from Boehringer);
- Clal 8mer (sequence CATCGATG, from New England Biolabs);
- Xhol 8mer (sequence CCTCGAGG, from New England Biolabs).
Ligation reactions are performed as described in 1.5.5 in 10 microl buffer with 12U T4 DNA ligase, the DNA fraction is extracted and precipitated, redissolved in 300 microl of the appropriate buffer containing respectively 360U of Asp718, 200U of Clal or 240U of Xhol for 16 hours at 37° C. The DNA fractions from three reaction mixtures are separately extracted, precipitated and the DNA is self-annelead (1.5.5) with 1U ligase in 100 microl buffer for 16 hours. After extraction and precipitation, each DNA fraction is redissolved in 5 microl water and used to transform 100 microl of competent DH5 E.Coli cells. Among the tetracycline positive colonies of each trasformation series, one colony per series is selected by miniprep analysis for having the correct restriction pattern and named, respectively, RSVuPA-Tet-Asp, RSVuPA-Tet-Cla and RSVuPA-Tet-Xhol (Fig. 17).

E6.3 - Preparation of GASGAs expression vectors

The uPA receptor binding region (GFD) of human uPA is destroyed by opening the plasmids described in E6.1 and .2 at the newly introduced site and filling the resulting protruding ends. Depending on the nucleotides added during the filling reaction, the result is either a 12bp insert (when all the nucleotides are added (see E6.3.1)) or a 6bp insert (when only the first nucleotide is added and the rest of the protruding end is digested by the E. coli DNA polymerase (see E6.3.2)). These GASGAs vectors maintain the uPA reading frame but the expressed protein misses the capability of binding to its cellular receptor.

E6.3.1 - Complete filling

1 microgram of each of the Asp 718 or Xhol modified plasmids described in E3.2 can be further modified by linearizing them at the inserted linker by digestion with 5U of the appropriate enzyme in 50 microl at 37° C (1.5.1) thus obtaining protruding ends of four nucleotides per side. The DNA can be extracted and precipitated (1.5.2 and 1.5.3) redissolved in 50 microl of the appropriate buffer (Maniatis et al) containing all the four nucleotides and treated with 5U of DNA Polymerase, Klenow fragment, at room temperature for 30 min. The obtained flush ended DNA can be extracted and precipitated as usual. A portion corresponding to 10% of this precipitate is self-ligated in 100 microl (1.5.5) with 6U DNA ligase for 16 hours.
After extraction and precipitation each DNA fraction can be redissolved in 5 microl water and used to transform 100 microl competent DH5 E.Coli cells. For each trasformation, among the tetracycline positive colonies, one can be selected by miniprep analysis for having the expected restriction pattern and named respectively (Fig. 18):
- RSV-SVRTD, from full filling of the Asp 718 site in RSVuPA-Tet-Asp and in which a new Sna BI site has been introduced;
- RSV-SSIED, from full filling of the Xhol site in RSVuPA-Tet-Xho and in which a new Pvul site has been introduced;

E6.3.2 - Partial filling and/or blunting

1 microgram of each of the Asp718 or ClaI linker modified plasmids described in E6.2.3 (RSVuPA-Tet-Asp and RSVuPA-Tet-Cla) are further modified by linearizing them at the inserted linker by digesting with 5U of the appropriate enzyme in 50 microl buffer (final volume) at 37°C (1.5.1) thus obtaining 4 or 2 nucleotides protruding ends. Then the DNA can be extracted and precipitated (1.5.2 and 1.5.3), redissolved in 50 microl of the appropriate buffer (Maniatis et al) containing only 2mM dGTP in the case of Asp718 filling or only 2mM each dATP and dTTP in the case of ClaI blunting and treated with 12U of E.Coli DNA Polymerase (DNase free; (Boerhinger)) at room temperature for 30 min. The obtained flush ended DNA can be extracted and precipitated as usual and 10% of the obtained precipitate is self-ligated with 6U DNA ligase in 100 microl of the proper buffer (1.5.5) for 16 hours. After extraction and precipitation, each DNA preparation is redissolved in 5 microl water and used to transform 100 microl competent DH5 E.Coli cells. Among the tetracycline positive colonies, a clone per transformation series is selected by miniprep analysis for having the expected restriction pattern and is named, respectively (Fig. 18):
- RSV-SAD, from partial filling of the Asp 718 site in RSVuPA-Tet-Asp, wherein a new EagI site has been introduced;
- RSV-SYD, from blunt ending of the ClaI site in RSVuPA-Tet-Cla and wherein a new NdeI site has been introduced.

E6.4 - Expression and analysis of "interrupted" GASGAs

Interrupted GASGAs expression vectors such as those described in E6.3 can be cotransfected with RSV-Neo in CHO cells and the RSV-Neo resistant clones selected and analyzed as described in E2.8. Activation of plasminogen by the "interrupted" GASGAs, immunological characteristics and uPA receptor binding properties parallel those obtained with RSVuPA-E0. Selected clones can be isolated and grown in fermenters while mantaining the selective pressure (c.f. E2.8).

E6.5 - Expression of "interrupted" GASGAs in episomal vectors

Episomal vectors, such as BPV based expression vectors (see E3), expressing "interrupted" GASGAs can be generated by substituting a BssHI/SacI fragment encompassing the uPA ORF from exon II to intron J (c.f. Fig. 3A) with an analogue fragment derived from the GASGAs expression vectors described in E6.3 by essentially following the preparation of GASGAs expressing, BPV derived plasmids described in E3. Such episomal vectors can be expressed and analyzed as described in E6.4.

E6.6 - Costruction of "interrupted" GASGAs expressing minigenes

A method to construct "interrupted" GASGAS expressing minigenes in which a trascription unit coding for a GASGA is placed under the trascriptional control of the uPA promoter or a derivative thereof is described in E5.4. For example, the preparation of GASGAs expressing, pXS derived plasmids (see E4) described in E5 can be essentially followed. Such minigenes uPA derived vectors can be expressed and analyzed as described in E6.4.

Preparations

P1. - Preparation of anti-GFD monoclonal antibodies

Monoclonal antibodies (MABs) specific to the GFD region of tcuPA/scuPA can be obtained by proper selection of the anti-uPA MABs coming from hybridomas whose spleen cell progenitors are from animals which were immunized with scuPA, tcuPA or ATF or a mixture thereof. A convenient probe for this selection is a peptidic fragment named $F_6$ which has an apparent molecular weight of about 6kD, an aminoacidic sequence corresponding to the uPA sequence from the residue of leucine at position 4 to the glutamic acid residue in position 43, and corresponds (includes or is a relevant portion of) the so-called GFD (growth factor-like domain) of uPA, i.e. an aminoacidic sequence highly homologous to a characteristic sequence of the epidermal growth factor. This area is thought to be responsible for the binding of uPA to its cellular surface receptors.

P1.1 - Production of monoclonal antibodies

Highly purified 54,000 Dalton urokinase 120,000 IU/Mg (PERSOLV [R] Lepetit) is used to immunize 7-week old female Balb/C mice. 10 Microgram of this urokinase in complete Freunds's adjuvant is injected into the peritoneum of the animals 60 days before fusion.

Another 10 microgram of urokinase is given to mice 30 days later. Ten days before fusion, anti-urokinase antibody titre is measured by conventional ELISA method in blood samples taken from the tail veins and 5 days before fusion, a final booster of 10 microgram of urokinase is given endovenously only to those animals having an antibody titer higher than 1:10,000. Spleens are then removed on the day of the fusion and spleen cells (about $1 \times 10^8$) are fused with about $5 \times 10^7$ mouse myeloma NSO (subline of P3/NS1/1Ag 4.1, ATCC accession number TIB 18) in 50% PEG 6000.

After fusion, which has been performed substantially following the method of C. Milstein and G. Köhler, the cells are resuspended in Dulbecco's Modified Eagle Medium (DMEM) supplemented with 10% fetal calf serum and HAT medium (0.1mM Hypoxantine, 0.25 mM Aminopterine, 0.017 mM Thymidine, Flow laboratories) and put onto 5 different Costar plates containing mouse macrophages (feeder layer).

The HAT medium is changed every 3 days and, from day 10 after fusion, the supernatants are tested in an ELISA immunoassay every 2-3 days for the presence of anti-urokinase monoclonal antibodies.

P1.2 - Screening of anti-urokinase monoclonal antibody-producing hybridomas by ELISA

According to a modification of the Perlman method (Immunochemistry, 8, 873 (1971)), flexible 96 well microtitre plates (Dynatech) are coated with 50 microliter/well of 20 microgram/ml solution of urokinase in phosphate buffered saline pH 7.2, (0.05 M sodium phosphate pH 7.2 which contains 0.5 M sodium chloride) and incubated for 1 h at room temperature. After a thorough washing phosphate buffered saline containing with 0.05% Tween 20, the wells are saturated with 3% bovine serum albumine in phosphate buffered saline, for 3 h at room temperature to avoid unspecific bindings. After washing carefully, 50 microliter of supernatant of hybridoma cultures (or the same volume of ascitic fluids) are added to the wells and left to react for two hours at room temperature. The plates are then washed and incubated with a 1:1000 dilution of rabbit-anti-mouse Ig conjugated with horseradish peroxidase (P161, DAKO), for 90 min at room temperature. After thorough washing, the plates are incubated with 200 microliter/well of 1 mg/ml solution of the chromogen o-phenylethylendiamine (SIGMA) in 0.1 M citrate buffer pH 6, 1.7 mM $H_2O_2$. The color is developed in 20 minutes and the optical density read at 492 nm against blanks with neither antigen nor antibody. Wells with color levels 4 times higher than the blanks are considered as positive.

P1.3 - Screening for anti-GFD monoclonal antibodies by immunoblotting

The clones which are positive in the above screening (P1.2) are further analyzed by an immunoblotting assay (Western blot) in the presence of scuPA, uPA, ATF and uPA fragments $F_{12}$, $B_{11}$ and $F_6$, which are obtained as described in P2 below. These reference compounds are run in parallel in a SDS-PAGE (containing 20% acrylamide) experiment (see 1.5.7) and then blotted on a nitro-cellulose membrane and analyzed against the supernatants of clones which were positive in the ELISA assay reported above.

Those MABs which bind scuPA/tcuPA, ATF and uPA fragment $F_6$ without binding fragment $F_{12}$ or $B_{11}$ are isolated and the producing clones are further cultivated to produce significant amounts of them. Two clones which produced MABs having GFD specificity (i.e. without binding capacity to the other uPA regions) are isolated and named CD1 and DC6 (see Tab. 3).

P1.4 - Mass cultivation of anti-urokinase producing hybridomas

Anti-urokinase antibody producing hybridomas are selected, cloned by limiting dilution, grown in mass cultures and injected into the peritoneum of Balb/C mice previously treated with 0.5 ml of Pristane (2,6,10,14-tetramethylpentadecane; Aldrich). Ascite fluids are collected 15 days later. The concentration of anti-urokinase monoclonal antibodies is in the range of 10-20 mg/ml. They are purified either by protein-A Sepharose or urokinase-Sepharose affinity chromatography.

P1.5 - Purification of anti-urokinase monoclonal antibodies by affinity chromatography on Sepharose-urokinase

A Sepharose-urokinase conjugate is prepared by reacting CNBr-activated Sepharose 4b (Pharmacia) high molecular weight urokinase (120,000 IU/Mg). The coupling efficiency is of about 20 mg urokinase/ml swollen gel. Ascite fluids obtained as above (1 ml per producing hybridoma) is then applied to the Sepharose-urokinase affinity column (1.6 cm x 15 cm) pre-equilibrated in 0.01 M sodium phosphate buffer pH 8.0. After rinsing with the same buffer, the selectively adsorbed anti-urokinase monoclonal antibodies are eluted with 0.1 M acetic acid pH 3.0. The antibody containing fractions are pooled and concentrated by ultrafiltration on PSDE 09005 Millipore membranes and stored frozen until use.

P1.6 - Evaluation of the purity and homogeneity of the monoclonal antibody preparations by gel-electrophoresis

The monoclonal antibody obtained above is submitted to SDS-PAGE (sodium dodecylphosphate polyacrylamide gel electrophoresis) according to the method described by W.K. Laemmli in Nature 227, 680 (1970). Only the bands corresponding to the heavy and the light chains of IgG are evident indicating that the above eluate contains pure immunoglobulins.

P2. - Preparation of uPA fragments $F_6$, $B_{11}$ and $F_{12}$

a) enzymatic cleavage of ATF and identification of the produced fragments ($F_6$, $B_{11}$ and $F_{12}$)

uPA amino-terminal fragment (ATF), (1.1 mg) prepared according to Stoppelli M.P. et al, Proc. Natl. Acad. Sci. USA, 1985, 82, 4939-4943, is treated with Staphylococcus aureus derived protease V8 (0.32 mg; Boehringer) in sodium phosphate buffer (50mM, pH 7.8) for 90 min at 37 C. Under these conditions the protease V8 cuts the ATF on the carboxylic side of glutamic acid residues. The enzymatic reaction is blocked by boiling the mixture 2 min and the proteic fragments so obtained are analyzed on SDS-PAGE (20% acrylamide) and compared to the original ATF. The band corresponding to ATF is no longer present, but three bands are observed with apparent molecular weight of 12.000, 11.000 and 6000, respectively, as determined by comparison with standards of given molecular weight.

b) affinity chromatography separation of fragments $F_6$, $B_{11}$ and $F_{12}$

The above obtained mixture is loaded on a 5B4-agarose matrix (an agarose modified matrix bearing a monoclonal antibody, named 5B4, which binds the high molecular weight urokinase without binding the low molecular form prepared according to conventional methods by linking MAB 5B4 with Sepharose (Pharmacia) as described by Corti A., Nolli ML., Soffientini A. and Cassani G. in Throm Haemostas 1986; 56, 219-224). This column was previously equilibrated with 0.15 M sodium chloride and 0.05 M sodium phosphate buffer pH 7.4 containing 1% polyethylenglycol (PEG) 6000.

## c) recovery of the unbound fraction

The equilibrating buffer is passed through the column and fractions are collected, U.V. monitored (280 nm) and pooled depending on their content. Two major pooled fractions are obtained, one which contains the uPA fragment with apparent molecular weight of 12,000 (which is named $F_{12}$) and another one with apparent molecular weight of 6000 (which is named $F_6$). These fragments are purified by ion exchange chromatography followed by desalting in reverse phase chromatography as described below under e).

## d) recovery of the bound fraction

The fraction bound to the matrix is then eluted with 1 M sodium chloride and 0.1 M acetic acid containing 1% PEG 6000. Fractions are collected, monitored by U.V. (280 nm) and pooled according to their content. A single main fraction is obtained which contain the uPA fragment with apparent molecular weight of 11,000 which is named $B_{11}$ and is purified as described below.

## e) purification of fragments $B_{11}$, $F_6$ and $F_{12}$

The pooled fractions which contains, separately, fragment $B_{11}$, $F_{12}$ or $F_6$ obtained above, are further purified and concentrated by ion exchange chromatography in an FPLC (fast protein liquid chromatography) system (Pharmacia Fine Chemicals, Sweden) using a Mono S HR5/5 column (strong cation exchanger based on beaded hydrophilic resin with narrow particle size distribution; the charged group is $-CH_2SO_3$) pre-equilibrated with 0.05 M sodium acetate buffer, pH 4.8.

The elution is made with a linear gradient of aqueous sodium chloride from 0 to 1 M at 60 ml/h in 45 min. The fractions containing, separately, fragments $B_{11}$, $F_6$ and $F_{12}$ are then desalted by submitting them to a reverse phase column chromatography as described in 1.6.3. The pure fragments are isolated from the fractions containing them by evaporating the solvent under reduced pressure.

## f) aminoacid sequencing of $F_6$, $B_{11}$ and $F_{12}$

The N-terminal sequence of fractions F12 and B11 was determined by automatic sequencer (Applied Biosystem, Model 470A Gas-Phase) and each peak resulted to be composed of a main fragment with minor contaminants, while fragment $F_6$ resulted to be a single peptide (Tab. 4). The fragment sequences show preferential cleavage by V8 at the carboxyl side of a glutamic acid residue. Fragment F12 and fragment B11 differ only for the clipping between position 52 and 53. The molecular weight of fragment $F_6$ corresponds to a peptide resulting by clipping the ATF at positions $Glu_3$ and $Glu_{43}$. Therefore V8 cuts a peptide in the GFD region and two peptides essentially corresponding to the so-called "kringle" region (differing only by a cleaved site).

Table 3

| mAB | scuPA | LMW | ATF | B11 | F12 | F6 |
|------|-------|-----|-----|-----|-----|-----|
| 5B4 | + | - | + | + | - | - |
| DC1 | + | - | + | - | - | + |
| CD6 | + | - | + | - | - | + |
| Recognition of different portion of uPA by monoclonal antibodies (mAB): 5B4 recognizes a conformational epitope on the kringle region, DC1 and CD6 recognize the GFD region. | | | | | | |
| Low molecular weight (LMW) refers to the 33kD degradation product of uPA comprising the aminoacid from 136 to 411. | | | | | | |

EP 0 308 716 A2

---
---

## Table 4

| F6   | 1   |     |     | 5   |     |     | 10  |     |     | 14  |
|------|-----|-----|-----|-----|-----|-----|-----|-----|-----|-----|
| a.a. | Leu | His | Gln | Val | Pro | Ser | Asn | --- | Asp | --- | Leu | Asn | Gly | Gly |
| uPA  | 4   |     |     | 8   |     |     | 13  |     |     | 17  |

Tab.4: N-terminal aminoacid sequence of fragment F6; uPA indicates the correspoding
positions in the uPA molecule; (cycles 8 and 10) did not detect any aminoacid; this is
interpreted as showing to Cys residues, since these residues are not directly
detectable by the HPLC system used.

---
---

**Claims**

1. Human urinary plasminogen activator derivatives having the region containing the Growth Factor-like domain altered.

2. A human urinary plasminogen activator derivative of claim 1 wherein the region between aminoacid 9 and aminoacid 50 is altered.

3. A human urinary plasminogen activator derivative claim 1 wherein the region between aminoacid 9 and aminoacid 45 is altered.

4. A human urinary plasminogen activator derivative of claim 1 wherein the region between aminoacid 19 and aminoacid 32 is altered.

5. A human urinary plasminogen activator derivative of claims 1, 2, 3 or 4 which is a deletion derivative of native scu-PA.

6. A compound according to claim 1 wherein the aminoacid sequence from aminoacid 9 to aminoacid 45 is replaced by a tyrosine unit.

7. A process for preparing a compound of claims 1, 2, 3, 4, 5 or 6 which comprises:
   a) chemically modifying a native scu-PA or uPA in the GFD region, or
   b) altering the uPA region coding for the GFD by deletion, insertion or site directed mutation.

8. A method according to claim 7 which comprises inserting into a suitable animal cell an expressing vector containing a uPA coding region of genomic origin with a deletion in the region coding for the protein GFD, and recovering a human urinary plasminogen activator of claims 1, 2, 3, 4, 5 or 6 after cultivation of the transformed cells.

9. A method according to claim 8 wherein the deletion is in the DNA region coding for the aminoacid sequence from 9 to 50.

10. A method according to claim 8 wherein the deletion is in the DNA region coding for the aminoacid sequence from 9 to 45.

11. A method according to claim 8 wherein the deletion is in the DNA region coding for the aminoacid sequence from 19 to 32.

12. A method according to claim 8 wherein the deletion is between bp 1027 and bp 1784 of the genomic DNA coding sequence.

13. A pharmaceutical composition which contains a compound of claims 1, 2, 3, 4, 5 or 6 in admixture with a pharmaceutically acceptable carrier

14. A compound of claims 1, 2, 3, 4, 5, or 6 for use as a medicine.

15. Use of a compound according to claims 1, 2, 3, 4, 5 or 6 for preparing a medicament for antithrombotic treatment.


Claims for the following Contracting States: ES, GR 1. A process for preparinga human urinary plasminogen activator derivatives having the region containing the Growth Factor-like domain altered, which comprises:
   a) chemically modifying a native scu-PA or uPA in the GFD region, or
   b) altering the uPA region coding for the GFD by deletion, insertion or site directed mutation, expressing the obtained modified coding vector into a suitable host cell and recovering the product therefrom.

2. A method according to claim 1 which comprises inserting into a suitable animal cell an expressing vector containing a uPA coding region of genomic origin with a deletion in the region coding for the protein GFD and recovering the obtained human urinary plasminogen activator after cultivation of the transformed cells.

3. A process according to claim 1 or 2 for preparing a compound which is a human urinary plasminogen activator derivative wherein the region between aminoacid 9 and aminoacid 50 is altered.

4. A process according to claim 1, 2 or 3 for preparing a compound which is a human urinary plasminogen activator derivative wherein the region between aminoacid 9 and aminoacid 45 is altered.

5. A process according to claim 1, 2, 3 or 4 for preparing a compound which is a human urinary plasminogen activator derivative wherein the region between aminoacid 19 and aminoacid 32 is altered.

6. A process according to claim 1, 2, 3, 4 or 5 for preparing a compound which is a human urinary plasminogen activator derivative which is a deletion or insertion derivative of native scu-PA.

7. A process according to claim 1, 2, 3, 4, or 5 for preparing a compound which is a human urinary plasminogen activator derivative wherein the aminoacid sequence from aminoacid 9 to aminoacid 45 is replaced by a tyrosine unit.

8. A method according to anyone of claims 1, 2, 3, 4, 5, 6 or 7 wherein the deletion is in the DNA region coding for the aminoacid sequence from 9 to 50.

9. A method according to anyone of claims 1, 2, 3, 4, 5, 6, or 7 wherein the deletion is on the DNA region coding for the aminoacid sequence 9 to 45.

10. A method according to anyone of claims 1, 2, 3, 4, 5, 6 or 7 wherein the deletion is in the DNA region coding for the aminoacid sequence from 19 to 32.

11. A method according to anyone of claims 1, 2, 3, 4, 5, 6 or 7 wherein the deletion is between bp 1027 and bp 1784 of the genomic DNA coding sequence.

12. Use of a compound according to claims 1, 2, 3, 4, 5 6 or 7 for preparing a medicament for antithrombotic treatment.

Fig. 1

Fig. 2

Fig. 3

EP 0 308 716 A2

HpaI 1

XmaIII 619
--- Aat II 769

SmaI 945

HindIII 6958

NdeI 6697

HIND III · BAM HI 69%

AsuII 1470

XbaI6132

Late genes

Early genes

BPV

TRANSFORMING FRAGMENT

EcoRI 2113

BaI I 5893

BstEII 2405

AvrII 2766

AFLII 5050

FSPI 3023 .

(BamHI  4450)

BstXI3381  KpnI 3455

pML2dx

amp^R

ORI

BamHI  EcoRI

PvuI

Psci

XmaIII  SalI

pBPV 230.8

Sarver et al. Mol.Cell.Biol. 5, 3507 (1985)

Fig. 4

Fig. 5

Fig. 6

Fig. 7

Fig. 8

EP 0 308 716 A2

Human uPA promoter sequence

```
                                                      GGG GAGGCGGACG TTGCAGTGAG CCGAGATTGT GCCACTGCAC TCCAGCCTGG
-2353
-2300 GTGACAGACC GAGACTCCGT CTCAAAAAAA AAAAAAAAAA AATATGGCTG GGCGTGGTGG CTCATGCCTG TAATCCCAGC ACTTTGGAAG GATGAGGTGG
-2200 GAGGACCCCT TGAACCCAGA AGCCCAGCAA AACCTTGTCT TTAAAAAAAA AAAAGAACTG TGCACAAAGA TTTCAGAGAG TGCTAAAGAT TAGCGCATGG
-2100 ATAAGGAAGT TCTGTGAAGA GTTGAAGTGC TAGGTGAAGA GGTGGCACGG CGGGAGGAGGG CGCGGAAGGG GAGAAAGGGT GTCACGCTTC ATAACGGTCT
-2000 CCAAACCTCT TTGTCCAGGA GGAAATGAAG TCATCTGTCC TCTCAGCAAT CAGCATGACA GCCTCCAGCC AAGTAACCCT GGAGTCATGA GAGCTGCTAG
-1900 GGGACCAACA TGAATCATGA CGCGCCCCCT GGGAATTTCC TGATTACTAA CCTGGGAGTT TCGGGGTAAG TCCTCAGGCT GCAGCATCTC TGTTTATCTT
-1800 CTGGTCACGT TTATTTACAA TTAATGGGTT CTCAAATCCA AACAAAACTG ACCACAGTCT TCTAGAGGAA GTAGCAAGGT TGGCTCTGAA GCCTATAGCA
-1700 TCTCTGACTC AGTCTGTCCC CTGGAAGGCT GGCAGCTCAG CAAGCACAGA AGTCTCTCCA GAAGACAGTG GGTCACCTGC CTCCCAAAAG CTGAAAGACT
-1600 AACTTGTAAT TTCCCCAGCA GGCAGCTGGG ATCCTGAGCC CTCGGCTGGG GCAGAGCAAA GGAGCCTTCC TCCTTCCTAC CTTCCTGGCA CTCTCCCTGC
-1500 CTTCCTTCTG TCACTCTCAG GTGGACCCAG ACCCAAGGTC CAGATTTGCA AGGCAGGAAA ATGCTGCAGG CCTAGGCTGG GAAAGGGCCC AAAGCCGCTA
-1400 GTGGATTGCT GGGACTCAGC CTCCTCCTTC CCACTAAGAG AGCGAGTCGT ACTGGGTTCA AAATGACCCC ANSCCCTGGT TTCTGACACT AGGGGAAAGA
-1300 GATGGGCGTG ACAGAATCAC AGAAQCCCTG CTATGTTCGT CCAAGTGTGC CCAGAGATGC GTGTGTGTGT GTGTGTATAC ACAAATGTCT GCTTATCCTC
-1200 AGGCAGGAAG GGTGGATNCA GTCATTTACA CATGGTCTGT TTTTCTGGAG GACAATTTTA TTTGATAAAC AATTGTTTCT ATCTGAATAG AATAAACAAG
-1100 GCTCTATGAT GAAGTAAAAC ACTAAATACA CATGCATTAA AAAATGCATA ATTATCTTTT TGGAATGGGC TATACAGAGA TGTGCTTTTT AACCTGTTAA
-1000 GAGTGTAAAA GGACAAACAG TGAAAARTRA TRCRTCCTCT TAQTTTGTCC TCCAGTCTCC CANTTCCTCT ACTCAGNGGT GAGNGAGNCT TCCACACCCC
-900 TCCAGAACCT CCACAGTTAG AACTGTCTAC ATGTTTCCCA TTGCTTTTAC TTTTATTCTT GCCTGCACAV ATAAATGAAT TGCTCCATTA TGGAAACTTC
-800 CCAAAACGCA TCCGCGTTAA CACTTCAATA GGAAGCACCA ACAGTTTATG CCCTAGGCTT TGTTCCCACA ATCCTGTAAC ATCATATCAC GACACCTAAC
-700 CCAATCCTTA TCAAGCCCTG TCAAAAACGG ACTTTAAACC AAGCTGCAAA TTTTCAGTAA TCTGGCCTTG CCTTTCCCCC TCTGATAGCA CCATCAAACA
-600 AACCCCCTTA CTGCCGAAAG CAATAAGCCC GCCTTTGTTC CATCCACTGG TTGTGTTGGT GATATCTGGG GACTGCCACT GAACAGACGC ACAGAGGGAG
-500 CCCCTACAGG CAGGGGTTTT TCTGTCTGTG CTTCTTGGGA GAGTATGTCT CGTACATTTG TCGCGTGATC AAGACTTCAC AGCTCCATCA GCTGCCGGCA
-400 AGGGGGTCTG AGGCAGTCTT AGGCAAGTTG GGGCCCAGCG GGAGAAGTTG CAGAAGAACT GATTAGAGGA CCCCAGGAGG CTTCAGAGCT GGGCGAGGTA
-300 GAGAGTCTCC TGTGCGCCTT CTCTCCTCTC TGCAATTCGG GGACTCCTTG CACTGGGCA GGCCCCCGGC AGGTGCATGG GAGGAAGCAC GGAGAATTTA
-200 CAAGCCTCTC GATTCCTCAG TCCAGACGCT GTTGGGTCCC CTCCGCTGGA GATCGCGCTT CCCCCAAATC TTTGTGAGCG TTGCCGAAGC ACGCGGGGTC
-100 CGGGTCGCTG AGCGCTGCAA GACAGGGGAG GGAGCCGGGC GGGAGAGGGA GGGGCGGCGC CGGGGCGGGC CCTGATATAG AGCAGGCGCC CCGGGTCGCA
+1 GCACAGTCGG AGACCGCAGC CCGGAGCCC
```

Fig. 9

puPA2

-2353  −1827      −1202         −573            +1  SmaI +29
SmaI   OxaNI      OxaNI         EcoRV

mRNA

puPA2/41

XhoI

puPA2/17

XhoI          OxaNI      Δ 625 bp      OxaNI

puPA2/254

XhoI          OxaNI         Δ 1290 bp            EcoRV

Fig. 10

EP 0 308 716 A2

Fig. 11

Fig. 12

Fig.14 Alternative way to construct GASGA-EO expression vectors

Fig. 15

Fig.16

Fig 17

| Plasmid | | | Modified site |
|---|---|---|---|

RSVuPA
```
        20              ScaI              26
TTC AAC AAG T              AC TTC TCC AAC        none
Ser Asn Lys T              yr Phe Ser Asn
```

RSV-SAD
```
        20              EagI              28
TTC AAC AAG TCG G        CC GAC TTC TCC AAC     Asp718(1)
Ser Asn Lys Ser A        la Asp Phe Ser Asn
```

RSV-SVRTD
```
        20            SnaBI               30
TTC AAC AAG TCG GTA CGT ACC GAC TTC TCC AAC     Asp718(2)
Ser Asn Lys Ser Val Arg Thr Asp Phe Ser Asn
```

RSV-SYD
```
        20              NdeI              28
TTC AAC AAG TCA T         AT GAC TTC TCC AAC    ClaI(3)
Ser Asn Lys Ser T         yr Asp Phe Ser Asn
```

RSV-SSIED
```
        20             PvuI               30
TTC AAC AAG TCC TCG ATC GAG GAC TTC TCC AAC     XhoI(2)
Ser Asn Lys Ser Ser Ile Glu Asp Phe Ser Asn
```

(1) partially filled and blunted site in the RSVuPA-Tet derivative
(2) completely filled site in the RSVuPA-Tet derivative
(3) blunted site in the RSV-uPA derivative

Fig.18

DNA and aminoacid sequence of interrupted GASGAs